(19) Europäisches Patentamt
European Patent Office
Office européen des brevets

(11) EP 0 309 401 B2

(12) NEUE EUROPÄISCHE PATENTSCHRIFT

(45) Veröffentlichungstag und Bekanntmachung des Hinweises auf die Entscheidung über den Einspruch:
**05.06.2002 Patentblatt 2002/23**

(45) Hinweis auf die Patenterteilung:
**26.10.1994 Patentblatt 1994/43**

(21) Anmeldenummer: **88810622.6**

(22) Anmeldetag: **13.09.1988**

(51) Int Cl.[7]: **C07D 401/14**, C07D 401/12, C07D 211/94, C09D 7/12, C08K 5/34, C08K 5/35

# (54) Stabilisierung einer Beschichtung mit sterisch gehinderten N-hydroxysubstituierten Aminen

Stabilization of coatings with N-hydroxy hindered amines

Stabilisation d'enduits par des amines N-hydroxy substituées avec empêchement stérique

(84) Benannte Vertragsstaaten:
**BE DE FR GB IT NL**

(30) Priorität: **21.09.1987 US 99418**
**21.09.1987 US 99412**
**21.09.1987 US 99413**

(43) Veröffentlichungstag der Anmeldung:
**29.03.1989 Patentblatt 1989/13**

(73) Patentinhaber: **Ciba Specialty Chemicals Holding Inc.**
**4002 Basel (CH)**

(72) Erfinder:
- **Winter, Roland A.E.**
  **Armonk New York 10504 (US)**
- **Galbo, James P.**
  **Hartsdale New York 10530 (US)**
- **Mar, Andrew**
  **Norwalk Connecticut 06851 (US)**
- **Behrens, Rudolf A.**
  **New Fairfield Connecticut 06812 (US)**
- **Malherbe, Roger François, Dr.**
  **CH-4058 Basle (CH)**

(56) Entgegenhaltungen:
**EP-A- 0 082 244**
**EP-A- 0 157 738**
**US-A- 4 315 848**
**US-A- 4 344 876**
**US-A- 4 426 471**
**US-A- 4 426 472**
**US-A- 4 465 757**
**US-A- 4 472 547**
**US-A- 4 547 537**
**US-A- 4 665 185**
**US-A- 4 668 721**

- **Ullmann, 5. Auflage, A18,S. 387 und 469;**
- **CRC Handbook of Chemistry and Physics, 61st. Edn. 1980-1981, F-97;**
- **Beyer/Walter, Lehrbuch der Organischen Chemie, 20. Auflage 1984, 243-244**
- **L.G. Wade, Organic Chemistry, 2nd Edn. 1991, 910**
- **"Handbook of Coatings Additives", Marcel Dekker Inc., New York 1987, pages 244-246**
- **Calbo (ed.), Handbook of Coatings Additives, Marcel Dekker Inc., New York 1987, 256-257**

Bemerkungen:
Die Akte enthält technische Angaben, die nach dem Eingang der Anmeldung eingereicht wurden und die nicht in dieser Patentschrift enthalten sind.

EP 0 309 401 B2

## Description

The instant invention relates to the stabilization of a wide variety of ambient curable or acid catalized thermosetting coating composition by the incorporation therein of N-hydroxy-substituted hindered amine light stabilizers. The invention further relates to the new compounds of this type of hindered amines.

Hindered amine light stabilizers are well known to be effective in stabilizing a host of organic substrates including polymers against the deleterious effects of light and oxygen. Such hindered amino light stabilizers have been used in the stabilization of hot-crosslinkable alkyd or acrylic metallic stoving lacquers (US 4,426,472) and in stabilizing acid-catalyzed stoving lacquers based on hot-crosslinkable acrylic, polyester or alkyd resins (US 4,344,876 and 4,426,471). The hindered amino light stabilizers of these patents do not possess structures having a hydroxyl group substituted directly on the hindered N-atom of the compound. EP-A-0082244 and EP-A-0157738 teach the preparation of some N-hydroxy substituted hindered amine derivatives and their ability to stabilise organic polymers against the deleterious effects of light and oxygen. Related hindered amine stabilizers have been utilized individually and in combination with ultra-violet light absorbers to improve the performance characteristics of coating systems. Notwithstanding such improvements, there still exists a need to further retard the photooxidation and photodegradation of coating systems and thereby provide increased effectiveness by maintaining the physical integrity of the coatings. Such effectiveness can be manifested by prevention of embrittlement, cracking, corrosion, erosion, loss of gloss, chalking and yellowing of the coating.

It has now been determined that the aforementioned improvements can be achieved by substitution on the hindered N-atom of the hindered amines with hydroxyl groups and the utilization of such derivatives in ambient curable and acid catalyzed thermosettings coating systems. In particular, the physical integrity of the coatings is maintained to a higher degree with significant reduction in loss of gloss and yellowing. Accordingly, the present invention relates to a stabilized ambient curing or acid catalyzed thermosetting coating composition containing an effective stabilizing amount of a hydroxyl substituted hindered amine compound containing the group of the formula

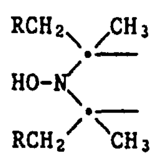

where R is hydrogen or methyl.

More particularly, the instant invention relates to the use of a hindered amine derivative corresponding to one of formulae A to M

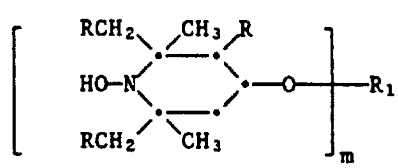

(A)

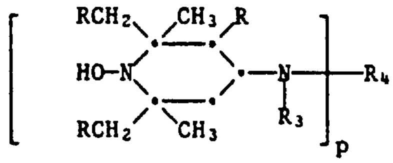

(B)

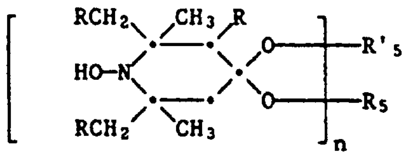

(C)

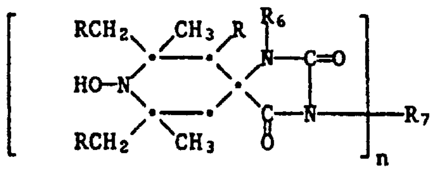

(D)

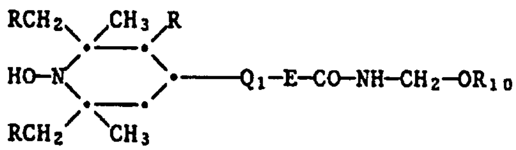

(E)

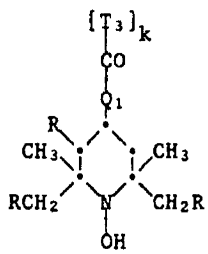

(F)

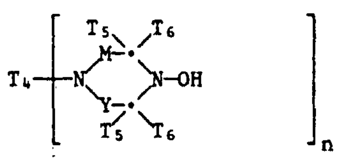

(G)

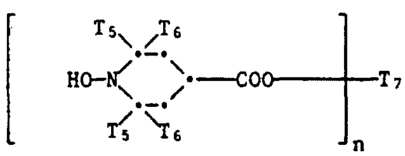

(H)

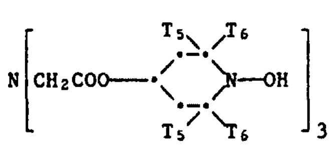

(I)

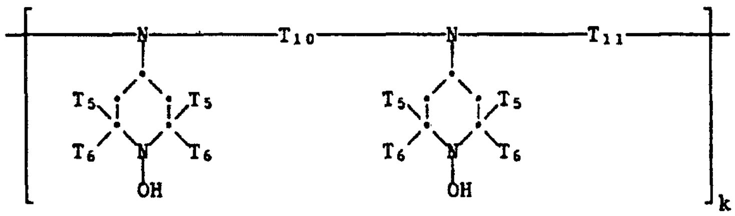

(J)

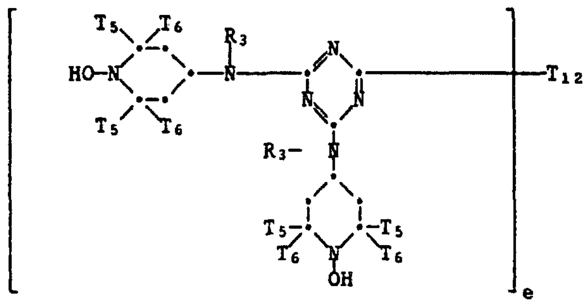

(K)

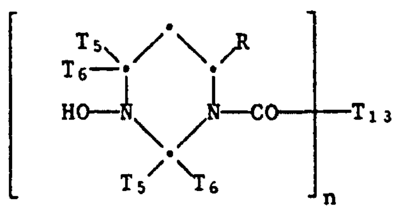

(L)

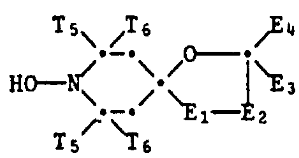

(M)

wherein
R is hydrogen or methyl,
m is 1-4, when m is 1,
$R_1$ is hydrogen, $C_1$-$C_{18}$ alkyl optionally interrrupted by one or more oxygen atoms, $C_2$-$C_{12}$ alkenyl, $C_6$-$C_{10}$ aryl, $C_7$-$C_{12}$ aralkyl, glycidyl, a monovalent acyl radical of an aliphatic, cycloaliphatic, araliphatic or aromatic carboxylic or carbamic acid, preferably an acyl radical of an aliphatic carboxylic acid having 2-18 C atoms, or a cycloaliphatic carboxylic acid having 5-12 C atoms or an aromatic carboxylic acid have 7-12 C atoms; or $R_1$ is a group

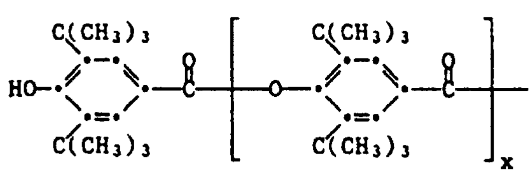

wherein x is 0 or 1, or is a group

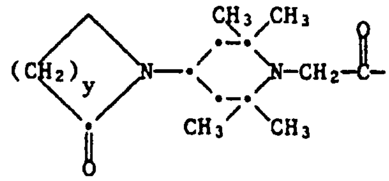

wherein y is 2-4;
when m is 2,
$R_1$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic or dicarbamic acid, preferably an acyl radical of an aliphatic dicarboxylic acid having 2-18 C atoms, of a cycloaliphatic or aromatic dicarboxylic acid having 8-14 C atoms, or of an aliphatic, cycloaliphatic or aromatic dicarbamic acid having 8-14 C atoms; or $R_1$ is a group

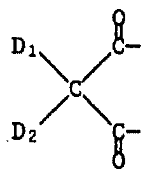

or

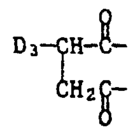

wherein $D_1$ and $D_2$ are independently hydrogen, an alkyl radical containing up to 8 carbon atoms, phenyl, benzyl or 3,5-di-t-butyl-4-hydroxybenzyl and $D_3$ is an alkyl or alkenyl radical containing up to 18 carbon atoms; when m is 3, $R_1$ is a triavalent acyl radical of an aliphatic, unsaturated aliphatic, cycloaliphatic, or aromatic tricarboxylic acid;
when m is 4, $R_1$ is a tetravalent acyl radical of a saturated or unsaturated aliphatic or aromatic tetracarboxylic acid including 1,2,3,4-butanetetracarboxylic acid, 1,2,3,4-but-2-enetetracarboxylic acid, and 1,2,3,5- and 1,2,4,5-pentane-tetracarboxylic acid;
p is 1 2 oder 3,
$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;
when p is 1,
$R_4$ is hydrogen, $C_1$-$C_{18}$ alkyl, $C_5$-$C_7$ cycloalkyl, $C_2$-$C_8$ alkenyl unsubstituted or substituted by cyano, carbonyl or carbamide group, aryl, aralkyl, or it is glycidyl, a group of the formula -$CH_2$-CH(OH)-Z or -CONH-Z wherein Z is hydrogen, methyl or phenyl; or $R_4$ is a group of formula I

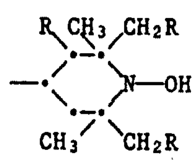

(I)

or

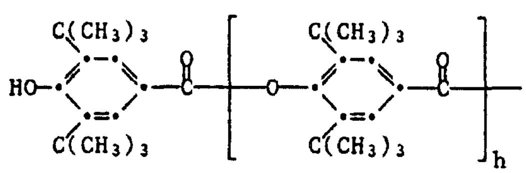

with h as 0 or 1;
or $R_3$ and $R_4$ together are alkylene of 4 to 6 carbon atoms or 2-oxoalkylene or the cyclic acyl radical of an aliphatic or aromatic 1,2- or 1,3-dicarboxylic acid,

when p is 2,
$R_4$ is $C_2$-$C_{12}$ alkylene, $C_6$-$C_{12}$ arylene, xylylene, a $-CH_2CH(OH)-CH_2-$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic acid or dicarbamic acid, or can be the group -CO-, or
$R_4$ is a group of formula II

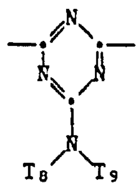

(II)

where $T_8$ and $T_9$ are independently hydrogen, alkyl of 1 to 18 carbon atoms or a group of formula I, or $T_8$ and $T_9$ together are alkylene of 4 to 6 carbon atoms or 3-oxapentamethylene, preferably $T_8$ and $T_9$ together are 3-oxapentamethylene;
when p is 3,
$R_4$ is 2,4,6-triazinetriyl,
n is 1 or 2 and
when n is 1,
$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;
when n is 2,
$R_5$ and $R'_5$ together are $(-CH)_2C(CH_2-)_2$;
$R_6$ is hydrogen, $C_1$-$C_{12}$ alkyl, allyl, benzyl, glycidyl or $C_2$-$C_6$ alkoxyalkyl;
when n is 1,
$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t-COO-Q$ or of the formula $-(CH_2)_t-O-CO-Q$ wherein t is 1 or 2, and Q is $C_1$-$C_4$ alkyl or phenyl; or
when n is 2,
$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH(OZ')-CH_2)_2-$ wherein Z' is hydrogen, $C_1$-$C_{18}$ alkyl, allyl, benzyl, $C_2$-$C_{12}$ alkanoyl or benzoyl;
$Q_1$ is $-N(R_8)-$ or -O-;
E is $C_1$-$C_3$ alkylene, the group $-CH_2-CH(R_9)-O-$ wherein $R_9$ is hydrogen, methyl or phenyl, the group $-(CH_2)_3-NH-$ or a direct bond;
$R_{10}$ is hydrogen or $C_1$-$C_{18}$ alkyl,
$R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH-(R_9)-OH$ wherein $R_9$ has the meaning defined above, or $R_8$ is a group of the formula I or a group of the formula

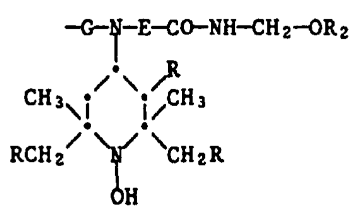

wherein G is $C_2$-$C_6$ alkylene or $C_6$-$C_{12}$ arylene, or $R_8$ is a group $-E-CO-NH-CH_2-OR_{10}$;
Formula F denotes a recurring structural unit of a polymer where $T_3$ is ethylene or 1,2-propylene, or is the repeating structural unit derived from an alpha-olefin copolymer with an alkyl acrylate or methacrylate;
preferably a copolymer of ethylene and ethyl acrylate, and where k is 2 to 100;
$T_4$ has the same meaning as $R_4$ when p is 1 or 2,

$T_5$ is methyl,
$T_6$ is methyl or ethyl, or $T_5$ and $T_6$ together are tetramethylene or pentamethylene or a mixture of said derivatives, preferably $T_5$ and $T_6$ are each methyl,
M and Y are independently methylene or carbonyl, preferably M is methylene and Y is carbonyl, and $T_4$ is ethylene where n is 2;
$T_7$ is the same as $R_7$, and $T_7$ is preferably octamethylene when n is 2,
$T_{10}$ and $T_{11}$ are independently alkylene of 2 to 12 carbon atoms, or $T_{11}$ is a group of formula II;
e is 2, 3 or 4 and
$T_{12}$ is a group $-N(R_5)-(CH_2)_d-N(R_5)-$
or

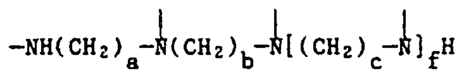

where a, b and c are independently 2 or 3, d is 2 to 10 and f is 0 or 1, preferably a and c are each 3, b is 2 and f is 1;
$T_{13}$ is the same as $R_4$ with the proviso that $T_{13}$ cannot be hydrogen when n is 1;
$E_1$ and $E_2$, being different, each are -CO- or $-N(E_5)-$, preferably $E_1$ is -CO- and $E_2$ is $-N(E_5)-$ where $E_5$ is hydrogen, $C_1$-$C_{12}$ alkyl or alkoxycarbonylalkyl of 4 to 22 carbon atoms;
$E_3$ is hydrogen, alkyl of 1 to 30 carbon atoms, phenyl, naphthyl, said phenyl or said naphthyl substituted by chlorine or by alkyl of 1 to 4 carbon atoms, or $E_3$ is phenylalkyl of 7 to 12 carbon atoms, or said phenylalkyl substituted by alkyl of 1 to 4 carbon atoms, and
$E_4$ is hydrogen, alkyl of 1 to 30 carbon atoms, phenyl, naphthyl or phenylalkyl of 7 to 12 carbon atoms, or
$E_3$ and $E_4$ together are polymethylene of 4 to 17 carbon atoms, or said polymethylene substituted by up to four alkyl groups of 1 to 4 carbon atoms, preferably methyl.

Of particular interest are coating compositions containing a hindered amine derivative of formula A, B, G-K or M wherein R is hydrogen and $T_5$ and $T_6$ are methyl.

In the structures A to M, if any substituents are $C_1$-$C_{18}$ alkyl, they are for example methyl, ethyl, n-propyl, n-butyl, sec-butyl, tert-butyl, n-hexyl, n-octyl, 2-ethylhexyl, n-nonyl, n-decyl, n-undecyl, n-dodecyl, n-tridecyl, n-tetradecyl, n-hexadecyl or n-octadecyl.

If $R_1$ is a monovalent acyl radical of a carboxylic acid, it is for example an acyl radical of acetic acid, stearic acid, salicylic acid, methacrylic acid, benzoic acid or β-(3,5-di-tert-butyl-4-hydroxyphenyl) propionic acid.

If $R_1$ is a divalent acyl radical of a dicarboxylic acid, it is for example an acyl radical of adipic acid, succinic acid, suberic acid, sebacic acid, phthalic acid, maleic acid, butyl-(3,5-di-tert-butyl-4-hydroxybenzyl)-malonic acid, or bicycloheptenedicarboxylic acid.

If $R_1$ is a divalent acyl radical of a dicarbamic acid, it is for example an acyl radical of hexamethylenedicarbamic acid or of 2,4-toluylene-dicarbamic acid.

If any substituents ar $C_5$-$C_7$ cycloalkyl, they are in particular cyclohexyl.

As $C_7$-$C_9$ aralkyl, $R_3$ is particularly phenethyl or above all benzyl.

As $C_2$-$C_{18}$ alkanoyl, $R_3$ is for example propionyl, butyryl, octanoyl, dodecanoyl, hexadecanoyl, octadecanoyl, but preferably acetyl; and as $C_3$-$C_5$ alkenoyl, $R_3$ is in particular acryloyl.

If $R_4$ is $C_2$-$C_8$ alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

If any substituents are $C_2$-$C_{12}$ alkylene, they are for example ethylene, propylene, 2,2-dimethylpropylene, tetramethylene, hexamethylene, octamethylene, decamethylene or dodecamethylene.

If any substituents are $C_6$-$C_{15}$ arylene, they are for example o-, m- or p-phenylene, 1,4-naphthylene or 4,4'-diphenylene.

As $C_6$-$C_{12}$ cycloalkylene, X is especially cyclohexylene.

If $R_5$ is $C_2$-$C_8$ alkylene or hydroxyalkylene, it is for example ethylene, 1-methyl-ethylene, propylene, 2-ethylpropylene or 2-ethyl-2-hydroxymethylpropylene.

As $C_4$-$C_{22}$ acyloxyalkylene, $R_5$ is for example 2-ethyl-2-acetoxymethylpropylene.

If any substituents are $C_2$-$C_6$ alkoxyalkyl, they are for example methoxymethyl, ethoxymethyl, propoxymethyl, tert-butoxyethyl, ethoxyethyl, ethoxypropyl, n-butoxyethyl, tert-butoxyethyl, isopropoxyethyl or propoxypropyl.

If $R_7$ is $C_3$-$C_5$ alkenyl, it is for example 1-propenyl, allyl, methallyl, 2-butenyl or 2-pentenyl.

As $C_7$-$C_9$ aralkyl, $R_7$ is in particular phenethyl or above all benzyl; and as $C_5$-$C_7$ cycloalkyl, $R_7$ is especially cyclohexyl.

If $R_7$ is $C_2$-$C_4$ hydroxyalkyl, it is for example 2-hydroxyethyl, 2-hydroxypropyl, 3-hydroxypropyl, 2-hydroxybutyl or 4-hydroxybutyl.

As $C_6$-$C_{10}$ aryl, $R_7$ is in particular phenyl, or alpha- or β-naphthyl which is unsubstituted or substituted by halogen or $C_1$-$C_4$ alkyl.

If $R_7$ is $C_2$-$C_{12}$ alkylene, it is for example ethylene, propylene, 2,2-dimethylpropylene, tetramethylene, hexamethylene, octamethylene, decamethylene or dodecamethylene.

If $R_7$ is $C_6$-$C_{12}$ arylene, it is for example o-, m- or p-phenylene, 1,4-naphthylene or 4,4'-diphenylene.

If Z' is $C_2$-$C_{12}$ alkanoyl, it is for example propionyl, butyryl, octanoyl, dodecanoyl or preferably acetyl.

As $C_5$-$C_7$ cycloalkyl, $R_8$ is in particular cyclohexyl.

As $C_6$-$C_{10}$ aryl, $R_8$ is particularly phenyl, or alpha- or β-naphthyl which is unsubstituted or substituted with halogen or $C_1$-$C_4$ alkyl. As $C_1$-$C_3$ alkylene, E is for example methylene, ethylene or propylene.

As $C_2$-$C_6$ alkylene, G is for example ethylene, propylene, 2,2-dimethylpropylene, tetramethylene or hexamethylene; and as $C_6$-$C_{12}$ arylene, G is o-, m- or p-phenylene, 1,4-naphthylene or 4,4'-diphenylene.

Preferred are coating compositions containing a hindered amine derivative of formula A wherein R is hydrogen, m is 1, 2 or 4 and when m is 1, $R_1$ is an acyl radical of an aliphatic carboxylic acid having 12-18 C atoms or of an aromatic carboxylic acid having 7-15 C atoms and
when m is 2, $R_1$ is a divalent acyl radical of an aliphatic dicarboxylic acid having 4-18 C atoms or of an aromatic dicarboxylic acid having 8-14 C atoms or $R_2$ is a group

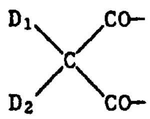

wherein $D_1$ is $C_1$-$C_8$ alkyl or 3,4-di-tert.butyl-4-hydroxybenzyl and $D_2$ is $D_1$ or hydrogen, and when m is 4, $R_1$ is tetravalent acyl radical of a butane- or pentanetetracarboxylic acid.

The following compounds are examples of hindered amine derivatives applicable for use in the invention:

1. 1,4-dihydroxy-2,2,6,6-tetramethylpiperidine
2. 1-hydroxy-4-salicyloxy-2,2,6,6-tetramethylpiperidine
3. 1-hydroxy-4-methacryloyloxy-2,2,6,6-tetramethylpiperidine
4. 1-hydroxy-2,2,6,6-tetramethylpiperidin-4-yl 3,5-di-tert-butyl-4-hydroxyhydrocinnamate
5. di(1-hydroxy-2,2,6,6-tetramethylpiperidin-4-yl) sebacate
6. di-(1-hydroxy-2,3,6-trimethyl-2,6-diethyl-piperidin-4-yl) phthalate
7. di-(1-hydroxy-2,2,6,6-tetramethylpiperidin-4-yl n-butyl-(3,5-di-tert-butyl-4-hydroxybenzyl)malonate
8. hexane-1',6'-bis-(4-carbamoyloxy-1-hydroxy-2,2,6,6-tetramethylpiperidine)
9. N,N'-bis(1-hydroxy-2,2,6,6-tetramethylpiperidin-4-yl)hexamethylene-1,6-diacetamide
10. 4-(N-cyclohexylacetamido)-1-hydroxy-2,2,6,6-tetramethylpiperidine
11. 4-methacrylamido-1-hydroxy-2,2,6,6-tetramethylpiperidine
12. N-(1-hydroxy-2,2,6,6-tetramethylpiperidin-4-yl)-epsilon-caprolactam
13. N-(1-hydroxy-2,2,6,6-tetramethylpiperidin-4-yl)-succinimide
14. N-(1-hydroxy-2,3,6-trimethyl-2,6-diethyl-piperidin-4-yl)-maleimide
15. 8-aza-2,7,7,9,9-pentamethyl-8-hydroxy-1,4-dioxaspiro[4.5]decane
16. 9-aza-3-hydroxymethyl-3-ethyl-9-hydroxy-8,8,10,10-tetramethyl-1,5-dioxaspiro[5.5]undecane
17. 3-n-octyl-1,3,8-triaza-8-hydroxy-7,7,9,9-tetramethyl-spiro[4.5]decane-2,4-dione
18. 8-hydroxy-2,7,7,9,9-pentamethyl-2-hexyl-1-oxa-3,8-diazaspiro[4.5]decane-4-one
19. 3-hydroxy-2,24,4-tetramethyl-7-oxa-3,20-diazaspiro[5.1.11.2]heneicosan-21-one
20. 1,1'-ethylenebis-(4-hydroxy-3,3,5,5-tetramethylpiperazin-2-one)
21. 1,1'-sebacoyl-bis-(3-hydroxy-2,2,4,4,6-pentamethylhexahydropyrimidine)
22. N-hydroxy derivative of polycondensation product of 2,4-dichloro-6-tert-octylamino-s-triazine and 4,4'-hexamethylenebis-(amino-2,2,6,6-tetramethylpiperidine)
23. N,N',N"N"'-tetrakis[4,6-bis(butyl-(1-hydroxy-2,2,6,6-tetramethylpiperidin-4-yl)amino)-s-triazin-2-yl]-1,10-diamino-4,7-diazadecane
24. N-hydroxy derivative of polycondensation product of 2,4-dichloro-6-morpholino-s-triazine and 4,4'-hexamethylenebis-(amino-2,2,6,6-tetramethylpiperidine)
25. 15-n-octadecyl-7-hydroxy-7,15-diazaspiro[5.1.5.3]hexadecane-14,16-dione

26. 4-benzyloxy-1-hydroxy-2,2,6,6-tetramethylpiperidine
27. 3-hydroxy-2,2,4,4-tetramethyl-20-(2-lauryloxycarbonyl)ethyl-7-oxa-3,20-diazaspiro[5.1.11.2]-heneicosan-21-one
28. di-(1-hydroxy-2,2,6,6-tetramethylpiperidin-4-yl) phthalate
29. 1-hydroxy-2,2,6,6-tetramethylpiperidin-4-yl stearate
30. 1-hydroxy-2,2,6,6-tetramethylpiperidin-4-yl acrylate
31. 4-(4-tert.butylbenzoyloxy)-1-hydroxy-2,2,6,6-tetramethylpiperidine
32. (1-hydroxy-2,2,6,6-tetramethylpiperidin-4-yl)-4-hydroxy-3,5-di-tert.butylbenzoate
33. (1-hydroxy-2,2,6,6-tetramethylpiperidin-4-yl)4-(hydroxy-3,5-di-tert.butylbenzoyl)-3,5-di-tert-butylbenzoate
34. (1-hydroxy-2,2,6,6-tetramethylpiperidin-4-yl)[4-(2-oxazepin-1-yl)-2,2,6,6-tetramethylpiperidin-1-yl)-acetate
35. alpha,alpha'-di-(1-hydroxy-2,2,6,6-tetramethylpiperidin-4-oxy)-p-xylene
36. di-(1-hydroxy-2,2,6,6-tetramethylpiperidin-4-yl) diethylmalonate
37. N-n-butyl-N-(1-hydroxy-2,2,6,6-tetramethylpiperidin-4-yl)-4-hydroxy-3,5-di-tert.butylbenzamide
38. tetrakis (1-hydroxy-2,2,6,6-tetramethyl-4-piperidyl) 1,2,3,4-butanetetracarboxylate
39. di-(1-hydroxy-2,2,6,6-tetramethylpiperidin-4-yl) n-butylmalonate
40. di-(1-hydroxy-2,2,6,6-tetramethylpiperidin-4-yl) isophthalate
41. di-(1-hydroxy-2,2,6,6-tetramethylpiperidin-4-yl) 2-(4-hydroxy-3,5-di-tert.butylbenzyl)-n-butylmalonate

The coating composition of this invention may be ambient curing or acid catalyzed hot curable systems, depending form the type of binder resins in the composition.

Resins for ambient curing coatings may be, for example, alkyd resins, thermoplastic acrylic resins, acrylic alkyd resins or polyester resins, or said resins modified with silicones, isocyanates, epoxides, isocyanurates, ketimines or oxazolidines. The resins may be esters of cellulose such as nitrocellulose or cellulose acetobutyrate or the resins may be epoxide resins hardenable with polyamines or other hardeners.

Applicable alkyd, acrylic, polyester and epoxide resins are described in S. Paul's "Surface Coatings: Science and Technology" (1985) at pages 70-310. The unmodified and modified alkyd resins which can be stabilized in accordance with the invention, are the conventional resins which are used in trade sales, maintenance and automotive refinish coatings. For example, such coatings are based on alkyd resins, alkyd/acrylic resins and alkyd/silicon resins optionally crosslinked by isocyanates or epoxy resins.

In addition various acrylic lacquer coating compositions are disclosed in US 4,168,249. Other acrylic/alkyd resins with polyisocyanate additives are disclosed in US 4,471,083; and acrylic resins containing either pendant amino ester groups or glycidyl groups are described in US 4,525,521.

Resins for acid catalyzed thermosetting coatings may be, for example, hot crosslinkable acrylic, polyester, polyurethane, polyamide or alkyd resins. This implies mixtures of those resins or mixtures with crosslinking agents such as melamine resins.

The acrylic resin lacquers are the conventional acrylic resin stoving lacquers or thermosetting resins including acrylic/melamine systems which are described, for example, in H. Kittel's "Lehrbuch der Lacke und Beschichtungen", Vol. 1. Part 2, on pages 735 and 742 (Berlin 1972), "Lackkunstharze" (1977), by H. Wagner and H.F. Sarx, on pages 229-238, and in S. Paul's "Surface Coatings: Science and Technology" (1985).

The polyester lacquers are the conventional stoving lacquers described e.g. in H. Wagner and H.F. Sarx, op. cit., on pages 86-99.

The alkyd resin lacquers which can be stabilized in accordance with the invention, are the conventional stoving lacquers which are used in particular for coating automobiles (automobile finishing lacquers), for example lacquers based on alkyd/melamine resins and alkyd/acrylic/melamine resins (see H. Wagner and H.F. Sarx, op. cit., pages 99-123). Other crosslinking agents include glycoluril resins, blocked isocyanates or epoxy resins.

In their industrial uses, enamels with high solids content based on crosslinkable acrylic, polyester, urethane or alkyd resins are cured with an additional acid catalyst. Light stabilizers containing a basic nitrogen group are generally less than satisfactory in this application. Formation of a salt between the acid catalyst and the light stabilizer leads to incompatibility or insolubility and precipitation of the salt and to a reduced level of cure and to reduced light protective action and poor resistance to moisture.

The N-hydroxy compounds of the present invention are less basic than the corresponding hindered amine compounds from which they are derived, yet still exhibit undiminished, indeed enhanced, light stabilization efficacy.

The ambient curing coatings as well as the acid catalyzed hot curable coatings stabilized in accordance with the invention are suitable both for metal finish coatings and solid shade finishes, especially in the case of retouching finishes. The lacquers stabilized in accordance with the invention are preferably applied in the conventional manner by two methods, either by the single-coat method or by the two-coat method. In the latter method, the pigment-containing base coat is applied first and a covering coat of clear lacquer applied over it.

The amount of hindered amine derivative employed is 0.1 to 10 % by weight, based on the solvent-free binder,

preperably 0.5 to 5 % by weight. The binders can be dissolved or dispersed in customary organic solvents or in water or can be solvent-free.

When used in two-coat finishes, the hindered amine derivative can be incorporated in the clear coat or both in the clear coat and in the pigmented base coat. In the manufacture of acrylic modified alkyd resins or acrylic resins, polymerizable hindered amine derivatives can be polymerized into the resin. The incorporation into the lacquer binder can also, however, be effected via polycondensation in the manufacture of the alkyd or polyester resins. In this cases, there is the additional advantage that the light stabilizers cannot be removed by extraction or migration so that their action is very prolonged.

To attain maximum light stability, the concurrent use of other conventional light stabilizers can be advantageous. Examples are UV absorbers of the benzophenone, benztriazole, acrylic acid derivative, or oxalanilide type, or aryl-s-triazines or metal-containing light stabilizers, for example organic nickel compounds. In two-coat systems, these additional light stabilizers can be added to the clear coat and/or the pigmented base coat.

If such combinations are employed, the sum of all light stabilizers is 0.2 to 20 % by weight, preferably 0.5 to 5 % by weight, based on the film-forming resin.

Examples of the UV absorbers which may be used in the instant compositions in conjunction with the aforementioned hindered amine compounds are:

(a) 2-(2'-Hydroxyphenyl)-benzotriazoles, for example the 5'-methyl-, 3',5'-di-tert-butyl-, 5'-tert-butyl-, 5'-(1,1,3,3-tetramethylbutyl)-, 5-chloro-3',5'-di-tert-butyl-, 5-chloro-3'-tert-butyl-5'-methyl-, 3'-sec-butyl-5'-tert-butyl-, 4'-octoxy-, 3',5'-di-tert-amyl derivative.

(b) 2-Hydroxy-benzophenones, for example, the 4-hydroxy-, 4-methoxy-, 4-octoxy-, 4-decyloxy-, 4-dodecyloxy-, 4-benzyloxy, 4,2',4'-trihydroxy- and 2'-hydroxy-4,4'-dimethoxy derivative.

(c) Acrylates, for example, alpha-cyano-β,β-diphenyl-acrylic acid ethyl ester or isoctyl ester, alpha-carbomethoxy-cinnamic acid methyl ester, alpha-cyano-β-methyl-p-methoxy-cinnamic acid methyl ester or butyl ester, alpha-carbomethoxy-p-methoxy-cinnamic acid methyl ester, N-(β-carbomethoxy-β-cyanovinyl)-2-methyl-indoline.

(d) Nickel compounds, for example, nickel complexes of 2,2'-thiobis[4-(1,1,3,3-tetramethylbutyl)-phenol], such as the 1:1 or 1:2 complex, optionally with additional ligands such as n-butylamine, triethanolamine or N-cyclohexyl-di-ethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketonoxime, nickel complexes of 1-phenyl-4-lauroyl-5-hydroxy-pyrazol, optionally with additional ligands.

(e) Oxalic acid diamides, for example, 4,4'-di-octyl-oxyoxanilide, 2,2'-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis-(3-dimethylaminopropyl)-oxalamide, 2-ethoxy-5-tert-butyl-2'-ethyloxanilide and its mixture with 2-ethoxy-2'-ethyl-5,4'-di-tert-butyloxanilide, and its mixtures of ortho- and paramethoxy- as well as of o-and p-ethoxy-disubstituted oxanilides.

(f) Hydroxyphenyl-5-triazines such as 2,6-bis-(2,4-di-methylphenyl)-4(2-hydroxy-4-octyloxyphenyl)-s-triazine or the corresponding 4(2,4-dihydroxyphenyl) derivatives.

Of particular value in the instant compositions are the benzotriazoles of high molecular weight and low volatility such as 2-[2-hydroxy-3,5-di(alpha,alpha-dimethylbenzyl)-phenyl]-2H-benzotriazole, 2-(2-hydroxy-3,5-di-tert-octylphenyl]-2H-benzotriazole, 2-(2-hydroxy-3-alpha,alpha-dimethylbenzyl-5-tert-octylphenyl)-2H-benzotriazole, 2-(2-hydroxy-3-tert-octyl-5,alpha,alpha-dimethylbenzylphenyl)-2H-benzotriazole, 2-(2-hydroxy-3,5-di-tert-amylphenyl)-2H-benzotriazole,2-[2-hydroxy-3-tert.butyl-5-(2-omega-hydroxy-octa(ethyleneoxy)-carbonyl)-ethylphenyl]-2H-benzotriazole, 2-[2-hydroxy-3-tert.butyl-5-(2-octyloxycarbonyl-ethyl)-phenyl]-benzotriazole, 2-(2-hydroxy-3-sec.dodecyl-5-methyl-phenyl)-benzotriazole, hexamethylene di[β-(3-tert.butyl-4-hydroxy-5-[2-benzotriazolyl]-phenyl)-propionate] and the 5-chloro compounds corresponding to each of the above named benzotriazoles.

Especially preferred is a benzotriazole UV absorber selected from the group consisting of 2-[2-hydroxy-3,5-di (alpha,alpha-dimethylbenzyl)-phenyl]-2H-benzotriazole, 2-(2-hydroxy-3,5-di-tert-octylphenyl)-2H-benzotriazole, 2-(2-hydroxy-3-alpha,-alpha-dimethylbenzyl-5-tert-octylphenyl)-2H-benzotriazole, 2-(2-hydroxy-3-tert-octyl-5,alpha, alpha-dimethylbenzylphenyl)-2H-benzotriazole, 2-(2-hydroxy-5-tert-octylphenyl)-2H-benzotriazole, 2-[2-hydroxy-3-tert-butyl-5-(2-(omega-hydroxy-octa(ethyleneoxy)-carbonyl)-ethylphenyl]-2H-benzotriazole, 2-(2-hydroxy-3,5-di-tert-amylphenyl)-2H-benzotriazole, 5-chloro-2-[2-hydroxy-3,5-di(alpha,alpha-dimethylbenzyl)-phenyl]-2H-benzotriazole, 5-chloro-2-(2-hydroxy-3,5-di-tert-butylphenyl)-2H-benzotriazole, 2-[2-hydroxy-3-tert.butyl-5-(2-octyloxycarbonylethyl)-phenyl]-5-chloro-2H-benzotriazole, 2-(2-hydroxy-3-sec.dodecyl-5-methyl-phenyl)-2H-benzotriazole and hexamethylene di[β-(3-tert.butyl-4-hydroxy-5-[2-benzotriazolyl]-phenyl)-propionate].

A preferred embodiment of the instant invention pertains to stabilized compositions comprising

(a) a resin system as defined hereinabove,

(b) an N-hydroxyl substituted 2,2,6,6-tetraalkylpiperidine compound,
(c) an UV absorber selected from the group consisting of the benzophenones, benzotriazoles, acrylic acid derivatives, organic nickel compounds, aryl-s-triazines and oxanilides.

Further ingredients which the enamels or coatings can contain are antioxidants, for example those of the sterically hindered phenol derivative type, phosphorus compounds, such as phosphites, phosphines or phosphonites, conventional hindered amine light stabilizers, plasticizers, levelling assistants, hardening catalysts, thickeners, dispersants or adhesion promoters.

A further preferred embodiment of the instant invention is a stabilized composition containing components (a), (b) and (c) described above which additionally contains as component (d) a phosphite or phosphonite.

The amount of phosphite or phosphonite (d) which is used in the instant compositions is from 0.05 to 2 % by weight, preferably from 0.1 to 1 % by weight, based on the film forming resin. In two-coat systems, these stabilizers may be added to the clear coat and/or base coat.

Typical phosphite and phosphonites include triphenyl phosphite, diphenylalkyl phosphites, phenyldialkyl phosphites, tri-(nonylphenyl)-phosphite, trilauryl phosphite, trioctadecyl phosphite, di-stearyl-pentaerythritol diphosphite, tris-(2,4-di-tert.butylphenyl) phosphite, di-isodecylpentaerythritol diphosphite, di-(2,4-di-tert.-butylphenyl)-pentaerythritol diphosphite, tristearyl-sorbitol triphosphite, tetrakis-(2,4-di-tert.butylphenyl)-4,4'-diphenylylenediphosphonite.

The stabilizers are needed to impart greater retention of durability to the cured enamels (as measured by 20° gloss, distinction of image, cracking or chalking); the enamel should not yellow on curing and further color change on exposure to light should be minimized; the stabilizers should be soluble in the organic solvents normally used in coating applications such as methyl amyl ketone, xylene, n-hexyl acetate, alcohol and the like.

The instant hindered amine light stabilizers substituted on the hindered N-atom by a hydroxy moiety fulfill each of these requirements and provide individually or in combination with a UV-absorber outstanding light stabilization protection to the cured coatings.

The following examples describe the inventive use of substituted hindered amine derivatives in various ambient curable and acid catalyzed hot curable resin systems. Parts and percentages are by weight.

Example 1: Stabilization of a White, Two-Component Acrylic Urethane Gloss Enamel

A white acrylic urethane enamel is formulated as shown below.

| Component I | Parts |
|---|---|
| Acryloid® AU 608 (acrylic polyol from Rohm & Haas) | 83.5 |
| $TiO_2$ | 196.7 |
| Cellosolve Acetate | 97.3 |
| - Sand Mill - | |
| Acryloid® AU 608 | 291.4 |
| Flow Improver | 0.28 |
| Cellosolve Acetate | 155.5 |
| Component II | |
| Desmodur® N-100 (polyisocyanate from Mobay Corp) | 70.9 |
| Cellosolve Acetate | 101.7 |
| | 997.3 |

This material is spray applied at a dry film thickness of 35-50 µm onto Bonderite 40 cold rolled steel panels that had been previously primed with a commercial epoxy polyamide maintenance primer (Sherwin-Williams Tile Clad II). Prior to application, the indicated amount of additive (based on resin solids) is added to the paint.

After ambient storage for a period of 2 weeks, the coated panels are subjected to weathering for 1790 hours in a QUV tester. In this test, the samples are subjected to weathering for 8 hours in a humid atmosphere and UV light at 50°C and for 4 hours with no UV light at 40°C. The 60° gloss of each panel (ASTM D-523) is determined and is reported below.

| Additive | Conc. (% by wt.) | 60° Gloss |
|---|---|---|
| A | 1.0 | 27 |

(continued)

| Additive | Conc. (% by wt.) | 60° Gloss |
|---|---|---|
| Compound No. 5 | 1.0 | 48 |
| A = bis(1,2,2,6,6-pentamethyl-4-piperidinyl)sebacate | | |

Example 2: Stabilization of Semi-Transparent Alkyd Stain

A semi-transparent linseed alkyd stain is formulated as shown below.

| Materials | Parts |
|---|---|
| Copolymer 186 (dehydrated castor oil from CasChem) | 27.3 |
| Mineral Spirits | 36.0 |
| Lecithin | 4.0 |
| Bentone SD-1 | 10.0 |
| Yellow Iron Oxide | 4.36 |
| Red Iron Oxide | 19.58 |
| Black Iron Oxide | 3.3 |
| Fungitrol® 11 | 2.0 |
| - Cowles Grind - | |
| Copolymer 186 | 66.3 |
| Beckosol® 10-051 (long oil alkyd from Reichold) | 115.0 |
| Linseed Oil | 20.3 |
| Mineral Spirits | 118.7 |
| 6 % Cobalt Naphthenate | 1.2 |
| 5 % Calcium Naphthenate | 2.16 |
| 18 % Zirconium Octoate | 1.2 |
| Ketoxime (antiskinning agent) | 1.0 |
| Xylol | 21.6 |
| Mineral Spirits | 273.4 |
| | 726.7 |

Pieces of 1.3 cm. x 20.3 cm x 30.5 cm Western Red Cedar panels having a fine radial cut are used. One half of each panel is coated with 3 coats of the unstabilized stain. An equal amount of stain containing 3 % (by weight on resin solids) of light stabilizers is applied to the other half of the panel in three coats. After storage at ambient temperatures for two weeks, the wood panels are exposed outdoors vertically facing south for a period of eight months. The 60° gloss retention of the stabilized and unstabilized section of the same panel are determined. Due to lack of homogeneity of wood substrates, the gloss retention of the same stain tends to differ from panel to panel. In this study, the unstabilized control stain is applied to every panel inasmuch as the differences in gloss retention between the stabilized and unstabilized portions of the panel allow for a better measure of the improvement in gloss due to the presence of the light stabilizer.

| Additive | 60 ° Gloss Value | | |
|---|---|---|---|
| | Unstabilized | Stabilized | Gloss Improvement |
| 3 % A | 21.4 | 20.3 | - |
| 3 % 29 | 27.5 | 69.8 | 42.3 |
| 1.5 %B + 1.5 %A | 16.9 | 32.4 | 15.5 |
| 1.5%B+1.5%29 | 23.9 | 66.7 | 42.8 |
| B = 5-(alpha,alpha-dimethylbenzyl)-2-(2-hydroxy-3,5-di-tert.butylpheny)-2H-benzotriazole | | | |

Example 3: Stabilization of a Medium Oil Alkyd Enamel

A commercially available medium oil alkyd enamel pigmented with non-leafing aluminium pigment and tinted light blue is stabilized with the indicated amount of UV absorber and hindered amine derivative (by weight of resin solids) and then spray applied onto cold rolled steed panels primed with an epoxy primer. After the coating is allowed to cure at room temperature for 2 weeks, the panels are exposed in a Xenon Arc Weatherometer for 840 hours. The 20 ° gloss retention (ASTM D-523-80) of the panels is determined.

| Additive | Conc. (% by wt.) | 20° Gloss Retention % |
|---|---|---|
| C/A | 3/2 | 22.7 |
| C/29 | 3/2 | 34.4 |
| C/39 | 3/2 | 31.9 |
| C = 2-[2-hydroxy-3-tert.butyl-5-(2-omega-hydroxy-octa-(ethyleneoxy)-carbonylethylphenyl)]-2H-benztriazole | | |

Example 4: Stabilization of a Nitrocellulose Lacquer

A commercially available white nitrocellulose lacquer (from Randolph Corp.) is stabilized with the indicated light stabilizers (by weight of resin solids) and spray applied onto epoxy primed cold rolled steel panels. After storage at room temperature for 2 weeks, the panels are exposed in a Xenon Arc Weatherometer for 1130 hours. The 60° gloss of the panels at that inverval is determined.

| Additive | Conc. (% by wt.) | 60° Gloss |
|---|---|---|
| C/A | 1.5/1.5 | 41 |
| C/39 | 1.5/1.5 | 70 |
| D/A | 1.5/1.5 | 36 |
| D/39 | 1.5/1.5 | 70 |
| D = 2-(2'-hydroxy-3'-(1-methylundecyl)-5'-methyl)-2H-benztriazole | | |

Example 5: Stabilization of a Blue Medium Oil Alkyd Enamel

A blue medium oil alkyd enamel is formulated as follows:

| Material | Wt. % |
|---|---|
| Aroplaz® 1445-M-50 (alkyd resin solution from NL Industries) | 66.6 |
| $TiO_2$ | 2.5 |
| Phthalocyanine Blue | 0.8 |
| Driers (Co, Pb, Mn) | 1.0 |
| Xylene | 29.1 |
| | 100.0 |

This material is spray applied to a thickness of 45 µm onto Bonderite 40 cold rolled steel panels which have been previously electrocoated with an epoxy ester (PPG Uniprine). The panels are allowed to cure at room temperature for 2 weeks and are then exposed outdoors at an angle of 45° facing south. After 9 months exposure, the 20° gloss retention of the coating is determined.

| Additive | Conc. (% by wt.) | 20° Gloss Retention % |
|---|---|---|
| Control (unstabilized) | - | 0 |
| E/5 | 2/1 | 69 |
| E = 2-(2'-hydroxy-3',5'-di-tert.butylphenyl)benztriazole | | |

Example 6: The following alkyd paint formulations are prepared.

| | White(W) | Parts Yellow(Y) | Blue(B) |
|---|---|---|---|
| Aroplaz® 1445 M-50 | 45.0 | 57.73 | 58.27 |
| $TiO_2$ | 45.0 | 20.82 | 4.26 |
| Irgalite® GS (yellow pigment from CIBA-GEIGY) | - | 7.3 | - |
| Phthalocyanine blue | - | - | 1.41 |
| Ketoxime (antiskinning agent) | 0.17 | 0.07 | 0.014 |
| Ionic antifloat compound | - | - | 0.05 |
| 24 % Pb as naphthenate | 0.94 | 1.30 | 1.30 |
| 6 % Co as naphthenate | 0.36 | 0.50 | 0.50 |
| 6 % Mn as naphthenate | 0.45 | 0.60 | 0.60 |
| Xylene | 35.0 | 48.4 | 45.1 |
| Mineral Spirits | 60.0 | 64.49 | 64.11 |

The formulations are stabilized with the indicated materials in the indicated concentrations (by weight on total resin solids) and sprayed onto cold rolled steel panels primed with an electrocoated epoxy primer. The coating is allowed to cure overnight at room temperature and the panels are then exposed in Florida at an angle of 45° South. 60° gloss, distinciton of image (DI) (Hunter Associates Apparatus) and color change based on Yellowness Index values are determined and tabulated below.

| Additive | Conc. (% by wt.) | Paint | Florida Exposure (Months) | | | | | | | | | |
|---|---|---|---|---|---|---|---|---|---|---|---|---|
| | | | 60° Gloss | | | | DI | | | | Color Change | |
| | | | 0 | 9 | 12 | 15 | 0 | 9 | 12 | 15 | 12 | 15 |
| - | - | W | 82 | 35 | 33 | 35 | 96 | 15 | 35 | 18 | 4.7 | 6.3 |
| E/5 | 2/1 | W | 82 | 55 | 49 | 45 | 96 | 74 | 66 | 53 | 3.5 | 5.2 |
| - | - | Y | 92 | 16 | 13 | 10 | 95 | 5 | 6 | 2 | 14 | 14 |
| E/5 | 2/1 | Y | 92 | 60 | 56 | 33 | 95 | 85 | 76 | 43 | 10 | 12 |
| - | - | B | 91 | 26 | 17* | - | 93 | 15 | 25 | - | 10 | - |
| E/5 | 2/1 | B | 91 | 84 | 75 | 54 | 93 | 82 | 78 | 68 | 2 | 3.4 |

* = cracking observed

Example 7: Stabilization of an Acrylic Alkyd Enamel Crosslinked with an Aliphatic Isocyanate Refinish Enamel

A commercially available light blue metallic acrylic alkyd enamel hardened with an aliphatic isocyanate is stabilized with the indicated amounts of additive (by weight on total resin solids) and then spray applied onto Bonderite 40 panels primed with an alkyd primer. After the coatings are aged at ambient temperature for 2 weeks, the panels are exposed outdoors at an angle of 5° for a period of 10 months. The 20° gloss retention of the panels is measured.

| Additive | Conc. (% by wt.) | 20° Gloss Retention % |
|---|---|---|
| C/F | 3/2 | 16 |
| C/36 | 3/2 | 27 |
| C/40 | 3/2 | 25 |
| C/39 | 3/2 | 24 |
| C/28 | 3/2 | 22 |
| F = bis(1,2,2,6,6-pentamethyl-4-piperidinyl)[[3,5-di-tert.-butylhydroxyphenyl]methyl]butylpropanedioate | | |

Example 8: Stabilization of a Thermoplastic Acrylic Lacquer

A commercially available light blue metallic thermoplastic acrylic lacquer is stabilized with the indicated amounts of additive (by weight on total resin solids) and then spray applied onto Bonderite 40 panels primed with an epoxy primer. After storage at ambient temperature for 2 weeks, the panels are exposed in a Xenon Arc Weatherometer for 1250 hours. The 60° gloss of the panels is determined.

| Additive | Conc. (% by wt.) | 60 ° Gloss |
|---|---|---|
| G/A | 2/2 | 11 |
| G/5 | 2/2 | 27 |
| G/32 | 2/2 | 24 |
| G/29 | 2/2 | 27 |
| G/28 | 2/2 | 31 |
| G/41 | 2/2 | 23 |
| G/39 | 2/2 | 39 |
| G/40 | 2/2 | 23 |
| G/36 | 2/2 | 23 |
| G/38 | 2/2 | 26 |
| G = 2-[2-hydroxy-3,5-di (alpha,alpha-dimethylbenzyl)-phenyl)]-2H-benzotriazole | | |

Example 9: Stabilization of an Acrylic Alkyd Refinish Enamel

A commercially available acrylic enamel pigmented with non-leafing aluminium pigment and tinted a light blue is stabilized with the indicated amounts of UV absorber and hindered amine (by weight of resin solids) and then spray applied onto Bonderite 40 panels primed with an alkyd primer. After the coating is allowed to cure at room temperature for 14 days, the panels are exposed outdoors at an angle of 5° S for a period of 10 months. The 20° gloss retention of the panels is measured.

| Additive | Conc. (% by wt.) | 20° Gloss |
|---|---|---|
| C/A | 3/2 | 18 |
| C/5 | 3/2 | 25 |
| C/28 | 3/2 | 26 |
| C/39 | 3/2 | 25 |
| C/40 | 3/2 | 28 |

Example 10: Stabilization of High Solids Acid-catalyzed, Thermoset Acrylic Resin Enamel

A high solids (50 % by weight) thermoset acrylic resin enamel, catalyzed by 0.5 % by weight of p-toluenesulfonic acid, based on the film-forming resin, is stabilized by the addition of various N-hydroxypolyalkylpiperidine derivatives.

The high solids thermoset acrylic resin enamel formulation (Acryloid® AT 400 from Rohm & Haas) is based on hydroxyethyl methacrylate, methyl methacrylate, styrene, butyl acrylate and butyl methacrylate and a melamine curing agent.

Pieces of steel sheeting 9 x 30 cm, coated with a primer based on polyester/epoxy resin, are then coated with a silver metallic base coat and finally with the clear finishing enamel. The base coat is sprayed onto the sheet to a thickness of about 20 µm and air dried for 3 minutes. The clear finishing enamel coat is then sprayed onto the sheet to a thickness of about 45 µm. After 15 minutes air-drying, the coated sheets are baked for 30 minutes at 250 °F (121 °C).

The stabilizers under test are added to the thermoset acrylic resin finishing enamel before the enamel is sprayed onto the base coated sheet.

After storage for 3 weeks in an air-conditioned room (23 °C/50 % relative humidity), the coated sheets are subjected to weathering for 1300 hours according to thest method ASTM G-53/77 in a QUV tester. In this test, the samples are subjected to weathering in repeated cycles for 4 hours in a humid atmosphere at 50°C and then for 8 hours under UV light at 70 °C. Subsequently, distinction of the image (DI) and the 20° gloss of the surface of the finishes are determined. High values for 20° gloss and DI are desired.

| QUV Exposure - 1300 hours | | |
|---|---|---|
| Stabilizer in clear coat | 20° Gloss | DI |
| Without Additive | 6 | 12 |
| 1 % Compound G + 2 % Compound 5 | 92 | 66 |
| 1 % Compound G + 2 % Compound 12 | 90 | 68 |

Example 11: In another test, the samples, prepared as in Example 10, after storage are subjected to accelerated weathering for 2230 hours in a Xenon arc (6500 Watt) Weather-Ometer.

The samples are exposed to repeated cycles for 102 minutes to the light source, then for 18 minutes to a water spray and light, at 60°C (black panel). The results are reported below. High values for 20° gloss and DI are desired.

| Xenon arc exposure | | |
|---|---|---|
| Stabilizer in clear coat | 20° Gloss | DI |
| Without Additive | 44 | 6 |
| 1 % Compound G + 2 % Compound 5 | 89 | 70 |
| 1 % Compound G + 2 % Compound 12 | 89 | 84 |

Example 12: Cracking Resistance

Samples described in Example 10 after being exposed in the QUV tester for 1300 hours are evaluated visually for cracking. The results are given below.

| Cracking after 1300 hours | |
|---|---|
| Stabilizer in clear coat | Appearance of the Enamel |
| Without Stabilizer | Deep cracks |
| 1 % Compound G + 2 % Compound 5 | No cracking |
| 1 % Compound G + 2 % Compound 12 | No cracking |

Example 13: Knoop Hardness and Enamel Appearance

Samples described in Example 10 after baking for 30 minutes at 121°C are stored for one week at room temperature. Knoop hardness values are determined on the baked enamel and the general appearance of the enamels is noted.

| Stabilizer in clear coat | Baked Coating Knoop Hardness | Appearance of Coating Before Baking |
|---|---|---|
| Without Stabilizer | 11.8 | Clear |
| 1 % Compound G + 2 % Compound 5 | 11.6 | Clear |
| 1 % Compound G + 2 % Compound 12 | 10.6 | Clear |

Example 14: Two thermoset acrylic enamels are formulated to include a benzotriazole UV-absorber and a hindered amine light stabilizer as described in Example 10.

The coated panels are exposed in the QUV tester, or in the Xenon Arc Weatherometer or in South Florida in an unheated black box at an angle of 5° to the sun. 20° gloss or distincition of image (DI) values are determined.

The thermoset acrylic enamels are based on a binder of 70 % of monomers such as hydroxyethyl acrylate, styrene, acrylonitrile, butyl acrylate and acrylic acid with 30 % of a melamine resin and as acid catalyst 0.5 % of p-toluene-sulfonic acid.

Enamel A is coated over a silver metallic base coat and Enamel B is coated over a white base coat.

| Enamel A with | 20° Gloss Values after Hours of QUV Exposure | | | | | | Hours of QUV Exposure Till First Cracking Observed |
|---|---|---|---|---|---|---|---|
| | 0 | 750 | 1300 | 1600 | 2350 | 3000 | |
| No Stabilizer | 95 | 82 | 8 | 2 | - | - | 900 |
| 1 % Compound G plus 2 % Compound 5 | 92 | 92 | 90 | 90 | 40 | 10 | 2700 |

| Enamel A with | 20° Gloss Values after Hours of Xenon Arc Exposure | | | | | | | | | | Hours of Xenon Arc Exposure Till First Cracking Observed |
|---|---|---|---|---|---|---|---|---|---|---|---|
| | 0 | 1400 | 1850 | 2500 | 2750 | 3300 | 4050 | 5100 | 5900 | 7400 | |
| No Stabilizer | 90 | 55 | 35 | 22 | 15 | 0 | - | - | - | - | 2700 |
| 1 % Compound G plus 2 % Compound 5 | 90 | 90 | 90 | 90 | 90 | 88 | 88 | 75 | 70 | 45 | >8500 |

| Enamel A with | Distinction of Image Values after Months of South Florida Exposure | | | |
|---|---|---|---|---|
| | 0 | 6 | 12 | 18 |
| No Stabilizer | 82 | 25 | 5 | 2 |
| 1 % Compound G plus 2 % Compound 5 | 85 | 82 | 75 | 60 |

| Enamel B with | Distinction of Image Values after Hours of QUV Exposure | | | | | | |
|---|---|---|---|---|---|---|---|
| | 0 | 200 | 1050 | 1450 | 1700 | 1840 | 2250 |
| No Stabilizer | 78 | 55 | 8 | 5 | - | - | - |
| 1 % Compound G plus 2 % Compound 5 | 88 | 88 | 88 | 88 | 88 | 88 | 60 |

Example 15: In order to determine whether cure is affected at either the 121°C normal bake temperature or at the 82°C automotive low bake repair temperature, Enamel A (as described in Example 14) containing various stabilizer combinations is baked over a silver base coat for 30 minutes at 121°C or for 30 minutes at 82°C.

The effectiveness of cure is assessed from Knoop hardness values, the higher numbers indicating greater hardness and hence cure.

| Enamel A containing 1 % by weight Compound G plus 2 % by weight of Test Compound | Knoop Hardness Values | |
|---|---|---|
| | Normal Bake | Low Bake Repair |
| Unstabilized | 11.5 | 11.5 |
| Compound A*) | 1-2 | - |
| Compound 5 | 11.5 | 7.0 |

* Compound A is di-(1,2,2,6,6-pentamethylpiperidin-4-yl) sebacate.

The instant compound 5 does not retard cure at the normal bake temperature and causes only slight insignificant retardation of cure at the automotive low bake repair temperature.

Example 16: The physical properties of Enamel A (as described in Example 14) coated over a silver metallic

base coat when cured for 30 minutes at 82°C, the low bake repair temperature, are completely satisfactory as is seen in the table below.

| Stabilizer | Knoop Hardness | Pendulum Hardness | Change in 20° Gloss Value after Humidity | Crosshatch Adhesion |
|---|---|---|---|---|
| Unstabilized | 7 | 70 | 20 | 4 |
| 1.5 % 5 + 1 % G | 7 | 85 | 2 | 5 |

The 20° gloss values are taken on coated panels conditioned for 96 hours in a constant humidity chamber at 38 C and 100 % relative humidity. A low change in 20° gloss value shows the coating is not adversely affected by humidity.

The cross-hatch type adhesion test involves using a multi-cut knife to prepare cross-hatches through the topcoat film and base coat on the panel. An acetate fiber adhesive tape is placed over the cross-hatch area and then is pulled off. A visual inspection of the amount of topcoat, if any, coming off with the tape as it is pulled gives a relative rating of the amount of delamination. A rating system of 5 indicating the absence of cross-hatch delamination to 0 indicating complete cross-hatch delamination is used.

It is clear that the low bake curing at 82°C for the acid catalyzed thermoset enamels containing the N-hydroxy hindered amine light stabilizers is completely adequate.

Example 17: The thermoset acrylic enamel of Example 14 including 0.5 % of p-toluenesulfonic acid is formulated to include varying concentrations of a benzotriazole UV-absorber and a hindered amine light stabilizer of the invention. The enamel is coated over a silver metallic base coat pursuant to the procedure in Example 14 and baking is conducted for 30 minutes at 121 °C normal bake temperature.

The coated panels are exposed in the QUV exposure apparatus and the time to 50 % loss of 20° gloss is determined.

| Compound | Conc. (% by wt.) | Time to 50 % Loss of 20 ° Gloss (hours) |
|---|---|---|
| unstabilized | - | 600 |
| G/5 | 2/1.5 | 3300 |
| G/22 | 2/1.5 | 2900 |
| G/29 | 2/1.5 | 2000 |
| G/37 | 2/1.5 | 1700 |
| unstabilized | - | 900 |
| C/5 | 3.5/1.5 | 4750 |
| C/5 | 3.5/1.5 | 5300 |
| C/28 | 3.5/1.5 | 4500 |
| C/29 | 3.5/1.5 | 5300 |
| C/31 | 3.5/1.5 | 3900 |
| C/35 | 3.5/1.5 | 4700 |
| C/39 | 3.5/1.5 | 5100 |
| C/40 | 3.5/1.5 | 5300 |
| unstabilized | - | 1000 |
| C/5 | 3/1 | 4700 |
| C/27 | 3/1 | 4100 |
| C/36 | 3/1 | 5200 |
| C/38 | 3/1 | 4600 |
| C/39 | 3/1 | 4200 |
| C/40 | 3/1 | 4600 |

Various N-hydroxy derivatives of hindered amines are known. For example, a substantial number of such derivatives are generically disclosed in US 4,590,231. The substituents on the 4-position include ether, ester, amino, dioxaspiro, diaza-dione, urea and triazine substituents, among others. These derivatives are identified as stabilizers in polyolefin compositions. Mono- and di-piperidinyl ester derivatives are likewise disclosed in JP-A 54-69162 for use in stabilizing urethane polymers. Other N-hydroxy derivatives are disclosed in J. Polym. Sci., Polymer Chem. Ed. 22, 277-281 (1984); Polym. Sci. Technol. 26, 35-47 (1984); Zh. Org. Khim. 6, 2365-2369 (1970); as well as in US 3,936,456,

US 4,404,302 and US 4,472,547.

Accordingly, it is a further object of the instant invention to identify a series of new N-hydroxy hindered amines having a broad range of stabilization performance characteristics.

These derivatives thus correspond to the formulae A' or B'

(A') 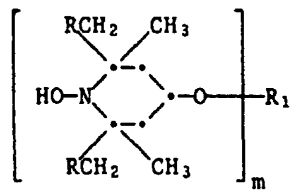

(B') 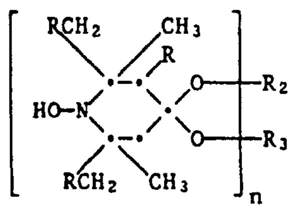

wherein R is hydrogen or methyl, preferably is hydrogen

m is 1-4;

when m is 1, $R_1$ is

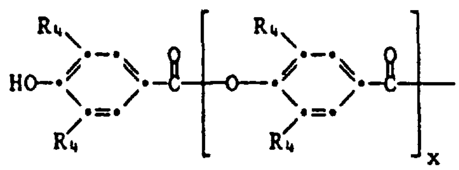

wherein x is 0 or 1 and $R_4$ independently are $C_1$-$C_8$alkyl, or

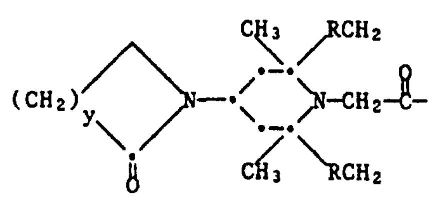

with y is 2-4;

when m is 2, $R_1$ is

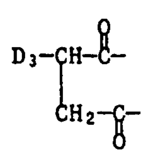 or 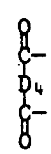

wherein $D_3$ is $C_1$-$C_{18}$-alkyl or $C_2$-$C_{18}$alkenyl, and $D_4$ is $C_5$-$C_{12}$cycloalkylene;

when m is 3, $R_1$ is a trivalent acyl radical of an aliphatic, unsaturated aliphatic, cycloaliphatic or aromatic tricarboxylic

acid having up to 20 C-atoms;
when m is 4, $R_1$ is a tetravalent acyl radical of a saturated or unsaturated aliphatic or aromatic tetracarboxylic acid having up to 20 C-atoms;
n is 1;
$R_2$ and $R_3$ are independently hydrogen, $C_1$-$C_{12}$alkyl, $C_2$-$C_{12}$alkenyl or $C_7$-$C_{12}$aralkyl.

In these formulae, representative alkyl groups include methyl, ethyl, n-propyl, n-butyl, tert-butyl, n-hexyl, n-octyl, 2-ethylhexyl, n-undecyl and n-dodecyl; aryl includes phenyl and naphthyl; aralkyl includes benzyl, alpha-methylbenzyl and phenethyl; and alkenyl includes 1-propenyl, allyl, methallyl, 2-butenyl, 2-hexenyl and 2-octenyl. $R_1$ as a trivalent acyl radical is, for example, an acyl radical of benzene-1,2,4-tricarboxylic acid. $R_1$ as a tetravalent acyl radical is, for example, an acyl radical of 1,2,3,4-butanetetracarboxylic acid, 1,2,3,4-but-2-enetetracarboxylic acid and 1,2,3,5- and 1,2,4,5-pentanetetracarboxylic acid. In the $R_1$ members when m is 2, malonates are preferred where $D_1$ is $C_1$-$C_8$alkyl or 3,5-di-t.butyl-4-hydroxybenzyl and $D_2$ is $D_1$ or hydrogen.

Preferred are the compounds of formula A' wherein
R is hydrogen, m is 1, 2 or 4 and
when m is 1, $R_1$ is a group

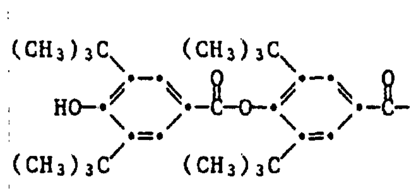

or

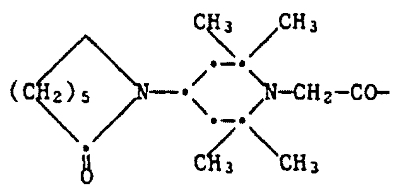

when m is 2, $R_1$ is a group

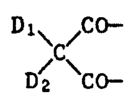

or

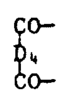

wherein $D_1$ is $C_1$-$C_4$alkyl or 3,5-di-t-butyl-4-hydroxybenzyl and $D_2$ is $D_1$ or hydrogen and $D_4$ is cyclohexylene,
and when m is 4, $R_1$ is the tetravalent acyl radical of a butanetetracarboxylic acid.

The N-hydroxy derivatives of formula A' und B' are generally prepared by oxidizing the corresponding N-H hindered amine with an appropriate peroxy compound such as hydrogen peroxide or tert-butyl hydroperoxide in the presence of molybdenum oxide or a metal carbonyl or metal oxide catalyst followed by reduction of the oxyl intermediate formed thereby to the desired N-hydroxy derivative, preferably by catalytic hydrogenation. Molybdenum (VI) is noted to increase the effeciency of the oxidation step. The reaction is preferably conducted in hydrocarbon solvents such as toluene. The starting materials needed to prepare the derivatives of this invention are items of commerce or can be prepared by known methods.

The derivatives are particularly effective in stabilizing organic materials primarily against the degradative effects of actinic stimuli. Such organic materials include polymeric materials such as the following polymers.

1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optioanlly can be crosslinked), for example high density polyethylene (HDPE), low density polyethylene (LDPE) and linear low density polyethylene (LLDPE).

2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).

3. Copolymers of monoolefines and diolefines with each other or with other vinyl monomers, such as, for example, ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/ acrylic acid copolymers and their salts (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene-copolymers, LDPE/EVA, LDPE/EAA, LLDPE/EVA and LLDPE/EAA.

3a. Hydrocarbon resins (for example $C_5$-$C_9$) and hydrogenated modifications thereof (for example tackyfiers).

4. Polystyrene, poly-(p-methylstyrene), poly-($\alpha$-methylstyrene).

5. Copolymers of styrene or $\alpha$-methylstyrene with dienes or acrylic derivatives, such as, for example, styrene/ butadiene, styrene/ acrylonitrile, styrene/alkyl methacrylate, styrene/maleic anhydride, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block copolymers of styrene, such as, for example, styrene/butadiene/ styrene, styrene/ isoprene/styrene, styrene/ethylene/butylene/ styrene or styrene/ethylene/propylene/styrene.

6. Graft copolymers of styrene or $\alpha$-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.

7. Halogen-containing polymers, such as polychloroprene, chlorinated rubbers, chlorinated or sulfochlorinated polyethylene, epichlorohydrin homo- and copolymers, polymers from halogen-containing vinyl compounds,as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/ vinyl acetate copolymers.

8. Polymers which are derived from $\alpha,\beta$-unsaturated acids and derivatives thereof, such as polyacrylates and polymethacrylates, polyacrylamide and polyacrylonitrile.

9. Copolymers from the monomers mentioned under 8) with each other or with other unsaturated monomers, such as, for instance, acrylonitrile/butadiene, acrylonitrile/alkyl acrylate, acrylonitrile/ alkoxyalkyl acrylate or acrylonitrile/ vinyl halogenide copolymers or acrylonitrile/alkyl methacrylate/butadiene terpolymers.

10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.

11. Homopolymers and copolymers of cyclic ethers, such as polyalkylene glycols, polyethylene oxide, polypropylene oxide or copolymers thereof with bis-glycidyl ethers.

12. Polyacetals, such as polyoxymethylene and those polyoxymethylenes which contain ethylene oxide as a comonomer; polyacetals modified with thermoplastic polyurethanes, acrylates or MBS.

13. Polyphenylene oxides and sulfides, and mixtures of polyphenylene oxides with polystyrene or polyamides.

14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).

15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene diamine and adipic acid; polyamides prepared from hexamethylenediamine and isophthalic or/and terephthalic acid and optionally an elastomer as modifier, for example poly-2,4,4,-trimethylhexamethylene terephthalamide or poly-m-phenylene isophthalamide. Further copolymers of the aforementioned polyamides with polyolefins, olefin copolymers, ionomers or chemically bonded or grafted elastomers; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).

16. Polyureas, polyimides and polyamide-imides.

17. Polyesters which are derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly-[2,2,-(4-hydroxyphenyl)-propane] terephthalate and polyhydroxybenzoates as well as block-copolyether-esters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or the cellulose ethers, such as methylcellulose; rosins and their derivatives.
27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.

The compounds of formula A' and B' alone or in combination with phenols can further be used in photographic layers as yellow dye light stabilizers, as cyan dye dark stabilizers, as antistain agents in magenta layers (especially for two-equivalent magenta couplers) and as thermal stabilizers for magenta couplers.
The following examples illustrate the preparation of these compounds.

Example 18: Di-(1-hydroxy-2,2,6,6-tetramethylpiperidin-4-yl) methylmalonate

A 4.5M solution of t-butyl hydroperoxide in toluene (25 ml, 113 mmol) is rapidly added to a mixture of 50.0 g (126 mmol) of di-(2,2,6,6-tetramethylpiperidin-4-yl) methylmalonate, 3.0 g of molybdenum trioxide, and 100 ml of toluene at 90 °C. Another portion of 4.5M t-butyl hydroperoxide in toluene (87 ml, 392 mmol) is added over 20 minutes. The reaction mixture is stirred for 3 hours at 85-90 C, then cooled and filtered. The filtrate is concentrated and crystallized from methanol to yield 20.5 g (38 %) of di-(1-oxyl-2,2,6,6-tetramethylpiperidin-4-yl) methylmalonate, an orange solid, m.p. 111-120°C.
A mixture of 20.3 g (47.6 mmol) of di-(1-oxyl-2,2,6,6-tetramethylpiperidin-4-yl) methylmalonate, 1.0 g of 5 % palladium on carbon, and 100 ml of dichloromethane is hydrogenated in a Parr apparatus (50 psi, ambient temperature). The catalyst is then filtered off, and the filtrate is diluted with heptane (50 ml). The dichloromethane-heptane solution is fractionally distilled (bath temperature 50°C) until the product begins to crystallize, then cooled and filtered to give 11.9 g (58 % yield) of the title compound, as a white crystalline solid, m.p. 153-155°C.

| | | | |
|---|---|---|---|
| Anal. Calcd. for $C_{22}H_{40}N_2O_6$ | C, 61.7; | H, 9.4; | N, 6.5. |
| Found | C, 61.5; | H, 9.2; | N, 6.6. |

The compounds in the following examples are prepared according to the procedure of Example 18 utilizing the appropriate starting materials.

Example 19: Di-(1-hydroxy-2,26,6-tetramethylpiperidin-4-yl) n-butylmalonate

m.p. 92-93°C

| | | | |
|---|---|---|---|
| Anal. Calcd. for $C_{25}H_{46}N_2O_6$ | C, 63.8; | H, 9.9; | N, 5.9; |

(continued)

| | | | |
|---|---|---|---|
| Found | C, 63.9; | H, 9.8; | N, 6.0. |

Example 20: Di-(1-hydroxy-2,2,6,6-tetramethylpiperidin-4-yl) diethylmalonate

m.p. 115-118° C

| | | | |
|---|---|---|---|
| Anal. Calcd. for $C_{25}H_{46}N_2O_6$ | C, 63.8; | H, 9.9; | N, 5.9; |
| Found | C, 63.9; | H, 10.0; | N, 5.9. |

Example 21: 1-Hydroxy-2,2,6,6-tetramethylpiperidin-4-yl 4-(4-hydroxy-3,5-di-tert-butylbenzoyloxy)-3,5-di-tert-butylbenzoate

m.p. 170-172°C

| | | | |
|---|---|---|---|
| Anal. Calcd. for $C_{39}H_{59}NO_6$ | C, 73.5; | H, 9.3; | N, 2.2; |
| Found | C, 72.7; | H, 9.2; | N, 2.3. |

Example 22 : 1-Hydroxy-2,2,6,6-tetramethylpiperidin-4-yl [4-(2-oxoazepin-1-yl)-2,2,6,6-tetramethylpiperidin-1-yl] acetate

m.p. 251-252°C

| | | | |
|---|---|---|---|
| Anal. Calcd. for $C_{26}H_{47}N_3O_4$ | C, 67.1; | H, 10.2; | N, 9.0; |
| Found | C, 67.1; | H, 9.9; | N, 8.9. |

Example 23: Di-(1-hydroxy-2,2,6,6-tetramethylpiperidin-4-yl) 2-(4-hydroxy-3,5-di-tert-butylbenzyl)-2-n-butylmalonate

m.p. 148-152°C

| | | | |
|---|---|---|---|
| Anal. Calcd. for $C_{40}H_{68}N_2O_7$ | C, 69.7; | H, 9.9; | N, 4.1; |
| Found | C, 69.9; | H, 10.0; | N, 4.2. |

Example 24: Tetrakis(1-hydroxy-2,2,6,6-tetramethyl-4-piperidyl) 1,2,3,4-butane-tetracarboxylate

m.p. 201-204°C

| | | | |
|---|---|---|---|
| Anal. Calcd. for $C_{44}H_{78}N_4O_{12}$ | C, 61.8; | H, 9.2; | N, 6.5; |
| Found | C, 61.5; | H, 9.2; | N, 6.4. |

Example 25: Di-(1-hydroxy-2,2,6,6-tetramethylpiperidin-4-yl) trans-1,4-cyclohexane-dicarboxylate

m.p. 185-192°C

| | | | |
|---|---|---|---|
| Anal. Calcd. for $C_{26}H_{46}N_2O_6$ | C, 64.7; | H, 9.6; | N, 5.8; |
| Found | C, 64.3; | H, 9.4; | N, 5.6. |

**Claims**

**1.** A stabilized ambient curing or acid catalyzed thermosetting coating composition containing an effective stabilizing amount of a hydroxyl substituted hindered amine compound containing the group

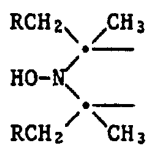

where R is hydrogen or methyl.

**2.** A composition of claim 1, which contains hindered amine derivative corresponding to one of the formulae A-M

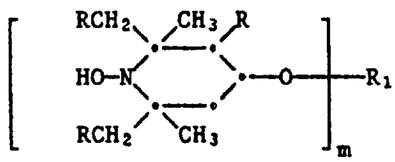

(A)

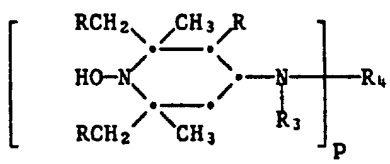

(B)

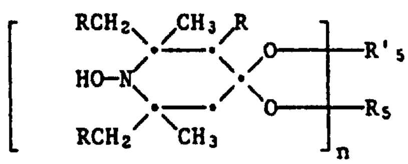

(C)

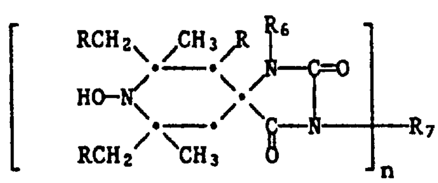

(D)

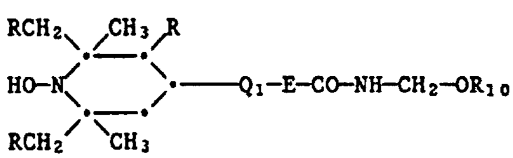

(E)

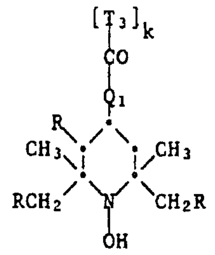

(F)

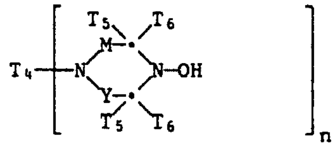

(G)

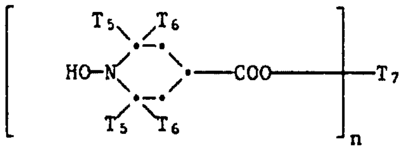

(H)

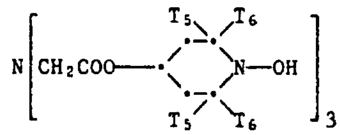

(I)

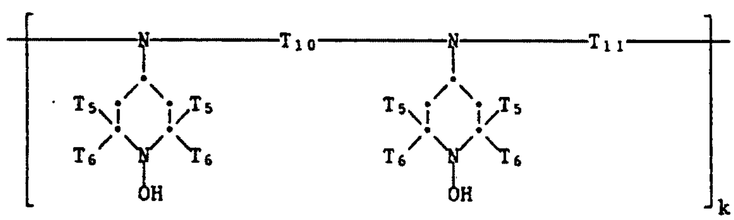

(J)

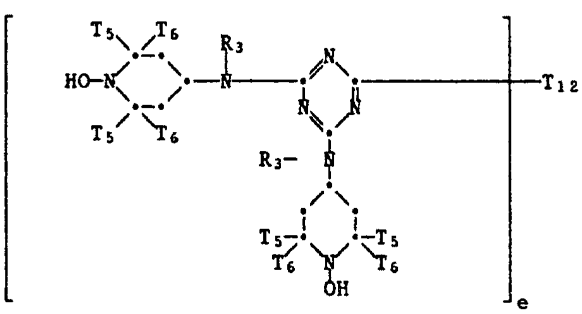

(K)

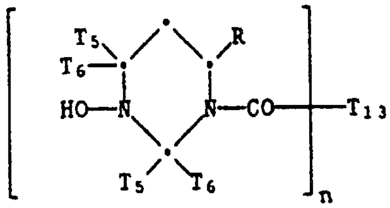

(L)

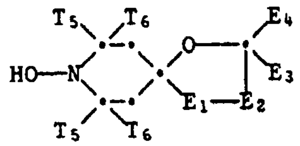

(M)

wherein
R is hydrogen or methyl,
m is 1-4,
when m is 1,
$R_1$ is hydrogen, $C_1$-$C_{18}$ alkyl optionally interrrupted by one or more oxygen atoms, $C_2$-$C_{12}$ alkenyl, $C_6$-$C_{10}$ aryl, $C_7$-$C_{12}$ aralkyl, glycidyl, a monovalent acyl radical of an aliphatic, cycloaliphatic, araliphatic or aromatic carboxylic or carbamic acid; or $R_1$ is a group

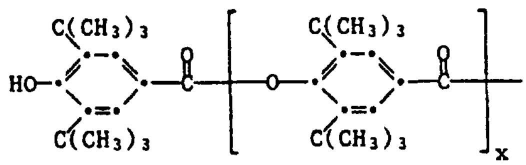

wherein x is 0 or 1, or is a group

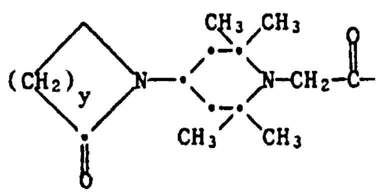

wherein y is 2-4;
when m is 2,
$R_1$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic or dicarbamic acid; or $R_1$ is a group

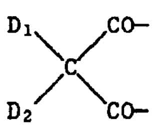

or

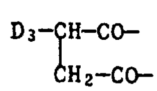

wherein $D_1$ and $D_2$ are independently hydrogen, $C_1$-$C_8$ alkyl, phenyl, benzyl or 3,5-di-tert.butyl-4-hydroxybenzyl and $D_3$ is an alkyl or alkenyl radical containing up to 18 carbon atoms;

when m is 3, $R_1$ is a trivalent acyl radical of an aliphatic, unsaturated aliphatic, cycloaliphatic, or aromatic tricarboxylic acid;
when m is 4, $R_1$ is a tetravalent acyl radical of a saturated or unsaturated aliphatic or aromatic tetracarboxylic acid;
p is 1, 2 or 3,
$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;
when p is 1,
$R_4$ is hydrogen, $C_1$-$C_{18}$ alkyl, $C_5$-$C_7$ cycloalkyl, $C_2$-$C_8$ alkenyl unsubstituted or substituted by cyano, carbonyl or carbamide group, aryl, aralkyl, or it is glycidyl, a group of the formula $-CH_2-CH(OH)-Z$ or -CONH-Z wherein Z is hydrogen, methyl or phenyl; or $R_4$ is a group of formula I

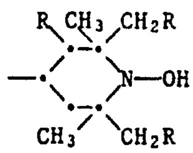 (I)

or a group of the formula

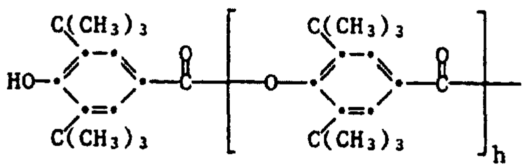

with h as 0 or 1;
or $R_3$ and $R_4$ together are alkylene of 4 to 6 carbon atoms or 2-oxoalkylene or the cyclic acyl radical of an aliphatic or aromatic 1,2- or 1,3-dicarboxylic acid,
when p is 2,
$R_4$ is $C_2$-$C_{12}$ alkylene, $C_6$-$C_{12}$ arylene, xylylene, a $-CH_2CH(OH)-CH_2-$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic acid or dicarbamic acid, or can be the group -CO- or
$R_4$ is a group of formula II

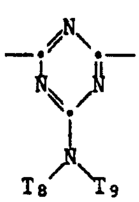 (II)

where $T_8$ and $T_9$ are independently hydrogen, alkyl of 1 to 18 carbon atoms or a group of formula I, or $T_8$ and $T_9$ together are alkylene of 4 to 6 carbon atoms or 3-oxapentamethylene;
when p is 3,
$R_4$ is 2,4,6-triazinetriyl,
n is 1 or 2 and
when n is 1,
$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;
when n is 2,
$R_5$ and $R'_5$ together are $(-CH_2)_2C(CH_2-)_2$;

$R_6$ is hydrogen, $C_1$-$C_{12}$ alkyl, allyl, benzyl, glycidyl or $C_2$-$C_6$ alkoxyalkyl;
when n is 1,
$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t$-COO-Q or of the formula $-(CH_2)_t$-O-CO-Q wherein t is 1 or 2, and Q is $C_1$-$C_4$ alkyl or phenyl; or
when n is 2,
$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH-(OZ')CH_2)_2-$ wherein Z' is hydrogen, $C_1$-$C_{18}$ alkyl, allyl, benzyl, $C_2$-$C_{12}$ alkanoyl or benzoyl;
$Q_1$ is $-N(R_8)-$ or -O-;
E is $C_1$-$C_3$ alkylene, the group $-CH_2-CH(R_9)-O-$ wherein $R_9$ is hydrogen, methyl or phenyl, the group - $(CH_2)_3$-NH- or a direct bond;
$R_{10}$ is hydrogen or $C_1$-$C_{18}$ alkyl,
$R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH(R_9)$ -OH wherein $R_9$ has the meaning defined above; or $R_8$ is a group of the formula I or a group of the formula

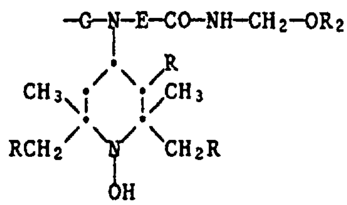

wherein G is $C_2$-$C_6$ alkylene or $C_6$-$C_{12}$ arylene, or $R_8$ is a group $-E-CO-NH-CH_2-OR_{10}$;
$T_3$ is ethylene or 1,2-propylene, or is the repeating structural unit derived from an alpha-olefin copolymer with an alkyl acrylate or methacrylate;
k is 2 to 100;
$T_4$ has the same meaning as $R_4$ when p is 1 or 2,
$T_5$ is methyl,
$T_6$ is methyl or ethyl, or $T_5$ and $T_6$ together are tetramethylene or pentamethylene;
M and Y are independently methylene or carbonyl;
$T_7$ is the same as $R_7$;
$T_{10}$ and $T_{11}$ are independently alkylene of 2 to 12 carbon atoms, or $T_{11}$ is a group of formula II;
e is 2, 3 or 4 and
$T_{12}$ is a group $-N(R_5)-(CH_2)_d-N(R_5)-$
or

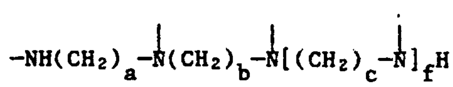

where a, b and c are independently 2 or 3, d is 2 to 10 and f is 0 or 1;
$T_{13}$ is the same as $R_4$ with the proviso that $T_{13}$ cannot be hydrogen when n is 1;
$E_1$ and $E_2$, being different, each are -CO- or $-N(E_5)-$, and $E_5$ is hydrogen, $C_1$-$C_{12}$-alkyl or $C_4$-$C_{22}$-alkoxycarbonylalkyl;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms, and
$E_4$ is hydrogen, alkyl of 1 to 30 carbon atoms, phenyl, naphthyl or phenylalkyl of 7 to 12 carbon atoms, or
$E_3$ and $E_4$ together are polymethylene of 4 to 17 carbon atoms, or said polymethylene substituted by up to four alkyl groups of 1 to 4 carbon atoms.

3. A composition according to claim 2, which contains a hindered amine derivative of formula A, B, G-K or M wherein R is hydrogen and $T_5$ and $T_6$ are methyl.

**4.** A composition according to claim 2, which contains a hindered amine derivative of formula A wherein R is hydrogen, m is 1, 2 or 4 and when m is 1, $R_1$ is an acyl radical of an aliphatic carboxylic acid having 12-18 C atoms or of an aromatic carboxylic acid having 7-15 C atoms and when m is 2, $R_1$ is a divalent acyl radical of an aliphatic dicarboxylic acid having 4-18 C atoms or of an aromatic dicarboxylic acid having 8-14 C atoms or $R_2$ is a group

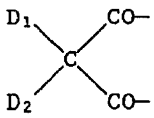

wherein $D_1$ is $C_1$-$C_8$ alkyl or 3,4-di-tert.butyl-4-hydroxybenzyl and $D_2$ is $D_1$ or hydrogen, and when m is 4, $R_1$ is tetravalent acyl radical of a butane- or pentane-tetracarboxylic acid.

**5.** A composition of claim 1, containing di(1-hydroxy-2,2,6,6-tetramethylpiperidin-4-yl) sebacate.

**6.** A composition of claim 1, containing 1-hydroxy-2,2,6,6-tetramethylpiperidin-4-yl stearate.

**7.** A composition of claim 1, containing di-(1-hydroxy-2,2,6,6-tetramethylpiperidin-4-yl) diethylmalonate.

**8.** A composition of claim 1, containing di-(1-hydroxy-2,2,6,6-tetramethylpiperidin-4-yl) isophthalate.

**9.** A composition of claim 1, containing tetrakis (1-hydroxy-2,2,6,6-tetramethyl-4-piperidyl) 1,2,3,4-butane-tetracarboxylate.

**10.** A composition of claim 1, containing the polycondensation product of 2,4-dichloro-6-tert-octylamino-s-triazine and 4,4'-hexamethylenebis(amino-1-hydroxy-2,2,6,6-tetramethylpiperidine).

**11.** A composition according to claim 1, wherein the hindered amine derivative is contained in an amount of 0.1 to 10 % by weight, based on resin solids.

**12.** A composition according to claim 1, which additionally contains a UV absorber selected form the group consisting of benzophenones, benztriazoles, acrylic acid derivatives, aryl-s-triazines, organic nickel compounds and oxanilides.

**13.** A composition according to claim 12, which contains a benzotriazole UV absorber selected from the group consisting of 2-[2-hydroxy-3,5-di(alpha,alpha-dimethylbenzyl)-phenyl]-2H-benzotriazole, 2-(2-hydroxy-3,5-di-tert-octylphenyl)-2H-benzotriazole, 2-(2-hydroxy-3-alpha,-alpha-dimethylbenzyl-5-tert-octylphenyl)-2H-benzotriazole, 2-(2-hydroxy-3-tert-octyl-5,alpha,alpha-dimethylbenzylphenyl)-2H-benzotriazole, 2-(2-hydroxy-5-tert-octylphenyl)-2H-benzotriazole, 2-[2-hydroxy-3-tert-butyl-5-(2-(omega-hydroxy-octa(ethyleneoxy)-carbonyl)-ethylphenyl]-2H-benzotriazole, 2-(2-hydroxy-3, 5-di-tert-amylphenyl)-2H-benzotriazole, 5-chloro-2-[2-hydroxy-3,5-di(alpha,alpha-dimethylbenzyl)-phenyl]-2H-benzotriazole, 5-chloro-2-(2-hydroxy-3,5-di-tert-butylphenyl)-2H-benzotriazole, 2-[2-hydroxy-3-tert.butyl-5-(2-octyloxycarbonylethyl)-phenyl]-5-chloro-2H-benzotriazole, 2-(2-hydroxy-3-sec.dodecyl-5-methyl-phenyl)-2H-benzotriazole and hexamethylene di[β-(3-tert.butyl-4-hydroxy-5-[2-benzotriazolyl]-phenyl)-propionate].

**14.** A coating composition according to claim 1, which is an ambient curable system based on an alkyd resin, thermoplastic acrylic resin, acrylic alkyd resin, polyester resin, or said resins modified with silicones, isocyanates, epoxides, isocyanurates, ketimines or oxazolidines, or the system is based on a cellulose ester or on an epoxide resin.

**15.** A coating composition according to claim 1, which is an acid catalyzed thermosetting system based on a hot crosslinkable acrylic, polyester, polyurethane, polyamide or alkyd resin.

**16.** A composition according to claim 1, which is an enamel for industrial finishes.

**17.** A composition according to claim 1, which is a refinishing enamel for automobiles.

**18.** A compound corresponding to the formulae A' or B'

(A') 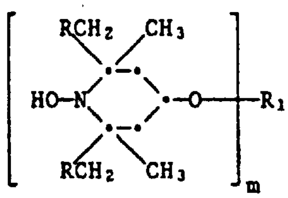

(B') 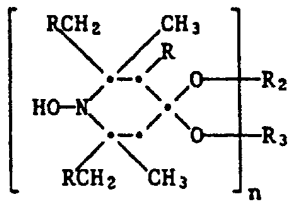

wherein R is hydrogen or methyl;

m is 1-4;

when m is 1, $R_1$ is

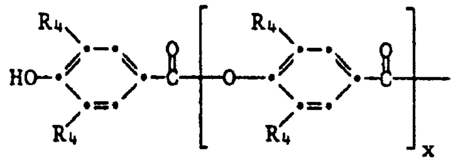

wherein x is 0 or 1 and $R_4$ independently are $C_1$-$C_8$ alkyl, or

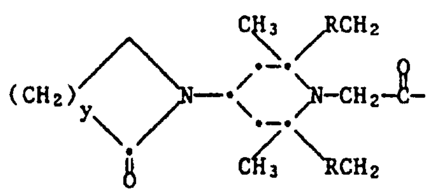

with y is 2-4;

when m is 2, $R_1$ is

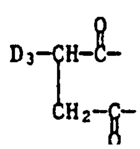

or

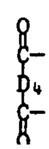

wherein

$D_3$ is $C_1$-$C_{18}$alkyl or $C_2$-$C_{18}$alkenyl, and $D_4$ is $C_5$-$C_{12}$cycloalkylene; when m is 3, $R_1$ is a trivalent acyl radical of an aliphatic, unsaturated aliphatic, cycloaliphatic or aromatic tricarboxylic acid having up to 20 C-atoms;

when m is 4, $R_1$ is a tetravalent acyl radical of a saturated or unsaturated aliphatic or aromatic tetracarboxylic acid

having up to 20 C-atoms;
n is 1,
when n is 1, $R_2$ and $R_3$ are independently hydrogen, $C_1$-$C_{12}$alkyl, $C_2$-$C_{12}$alkenyl or $C_7$-$C_{12}$aralkyl.

**19.** A compound of claim 18, wherein R is hydrogen.

**20.** A compound of formula A' of claim 18, wherein
R is hydrogen, m is 1, 2 or 4 and
when m is 1, $R_1$ is a group

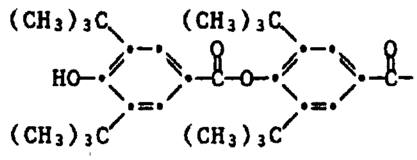

or

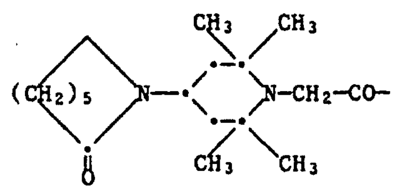

when m is 2, $R_1$ is a group

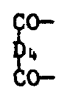

wherein $D_4$ is cyclohexylene,
and when m is 4, $R_1$ is the tetravalent acyl radical of a butanetetracarboxylic acid.

**21.** Di-(1-hydroxy-2,2,6,6-tetramethylpiperidin-4-yl) methylmalonate.

**22.** Di-(1-hydroxy-2,2,6,6-tetramethylpiperidin-4-yl) n-butylmalonate.

**23.** Di-(1-hydroxy-2,2,6,6-tetramethylpiperidin-4-yl) diethylmalonate.

**24.** Di-(1-hydroxy-2,2,6,6-tetramethylpiperidin-4-yl) 2-(4-hydroxy-3,5-di-tert-butylbenzyl)-2-n-butylmalonate.

**25.** Tetrakis(1-hydroxy-2,2,6,6-tetramethyl-4-piperidyl) 1,2,3,4-butanetetracarboxylate according to claim 18.

**26.** Use of a compound of claim 18 as stabilizer for organic materials against actinic degradation.

**27.** Use according to claim 26 as stabilizer for organic polymers.

**28.** Use according to claim 26 as stabilizer for photographic layers.

**Patentansprüche**

**1.** Eine stabilisierte, bei Umgebungstemperatur härtende oder säurekatalysierte, thermohärtbare Überzugszusammensetzung enthaltend eine wirksame stabilisierende Menge einer hydroxyl-substituierten gehinderten Aminverbindung, enthaltend die Gruppe der Formel

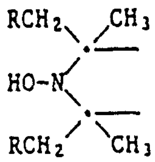

worin R Wasserstoff oder Methyl ist.

**2.** Zusammensetzung gemäß Anspruch 1, welche gehinderte Aminderivate entsprechend einer der Formeln A bis M enthält:

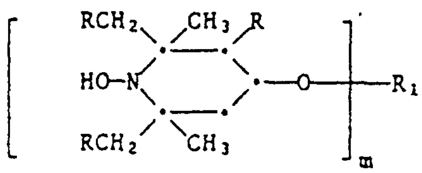

(A)

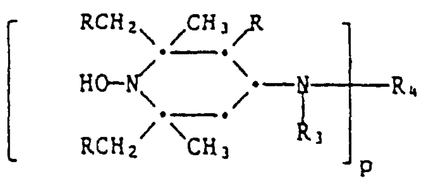

(B)

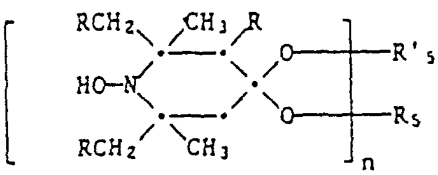

(C)

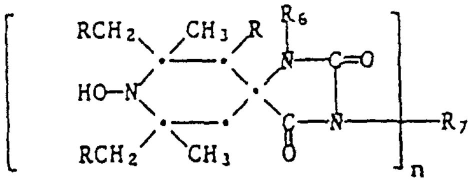

(D)

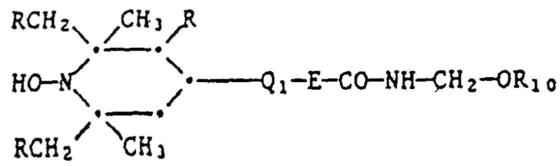

(E)

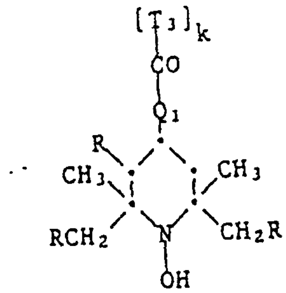

(F)

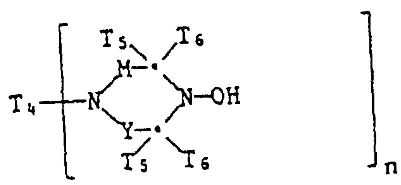

(G)

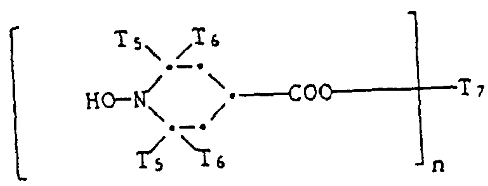

(H)

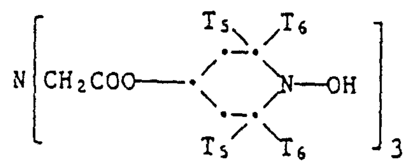

(I)

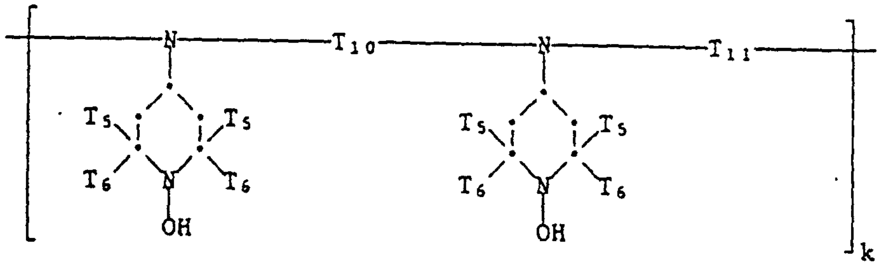

(J)

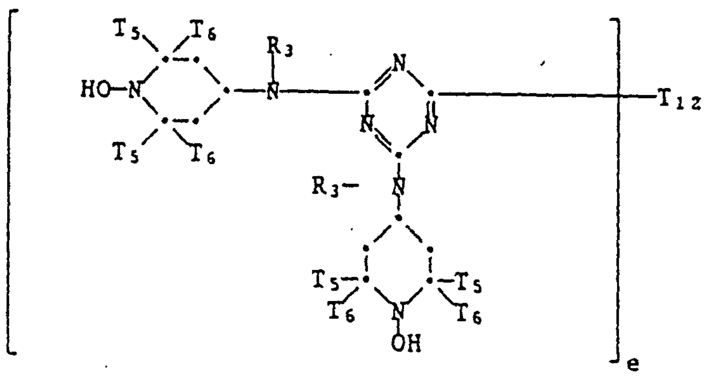

(K)

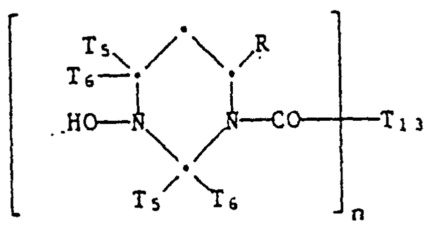

(L)

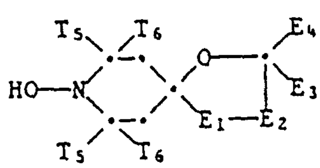

(M)

worin
R = Wasserstoff oder Methyl
n = 1 bis 4
wenn m = 1 ist,
ist $R_1$ Wasserstoff, $C_1$-$C_{18}$-Alkyl, gegebenenfalls unterbrochen durch ein oder mehrere Sauerstoffatome, $C_2$-$C_{12}$-Alkenyl, $C_6$-$C_{10}$-Aryl, $C_7$-$C_{12}$-Aralkyl, Glycidyl, ein einwertiger Acylrest einer aliphatischen, cycloaliphatischen, araliphatischen oder aromatischen Carbon- oder Carbamidsäure; oder $R_1$ ist eine Gruppe

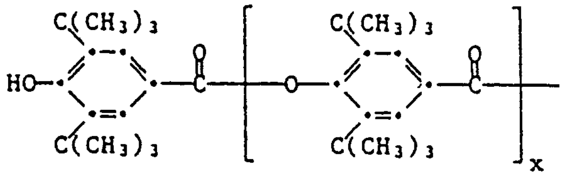

worin x = 0 oder 1 ist, oder eine Gruppe

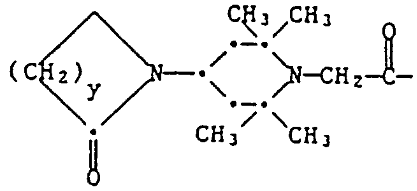

worin y = 2 bis 4 ist;
wenn m = 2 ist,
ist $R_1$ $C_1$-$C_{12}$-Alkylen, $C_4$-$C_{12}$-Alkenylen, Xylylen,
ein zweiwertiger Acylrest einer aliphatischen, cycloaliphatischen, araliphatischen oder aromatischen Dicarbonsäure oder Dicarbamidsäure; oder $R_1$ ist eine Gruppe

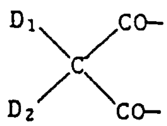

oder

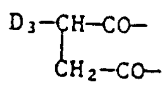

worin $D_1$ und $D_2$ unabhängig Wasserstoff, $C_1$ - $C_8$-Alkyl, Phenyl, Benzyl oder 3,5-Di-tert-butyl-4-hydroxybenzyl sind und $D_3$ ist ein Alkyl- oder Alkenylrest mit bis zu 18 Kohlenstoffatomen;
wenn m = 3 ist, ist $R_1$ ein dreiwertiger Acylrest einer aliphatischen, ungesättigten aliphatischen, cycloaliphatischen oder aromatischen Tricarbonsäure;
wenn m = 4 ist, ist $R_1$ ein vierwertiger Acylrest einer gesättigten oder ungesättigten aliphatischen oder aromatischen Tetracarbonsäure;
p = 1, 2 oder 3,
$R_3$ ist Wasserstoff, $C_1$-$C_{12}$-Alkyl, $C_5$-$C_7$-Cycloalkyl, $C_7$-$C_9$-Aralkyl, $C_2$-$C_{18}$-Alkanoyl, $C_3$-$C_5$-Alkenoyl oder Benzoyl;
wenn p = 1 ist,
ist $R_4$ Wasserstoff, $C_1$-$C_{18}$-Alkyl, $C_5$-$C_7$-Cycloalkyl, $C_2$-$C_8$-Alkenyl, unsubstituiert oder substituiert durch eine Cyano-, Carbonyl- oder Carbamidgruppe, Aryl, Aralkyl, oder es ist Glycidyl, eine Gruppe der Formel $-CH_2-CH(OH)-Z$ oder -CONH-Z, worin Z Wasserstoff, Methyl oder Phenyl ist; oder $R_4$ ist eine Gruppe der Formel I

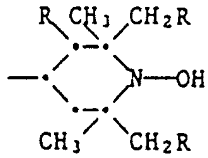

(I)

oder eine Gruppe der Formel

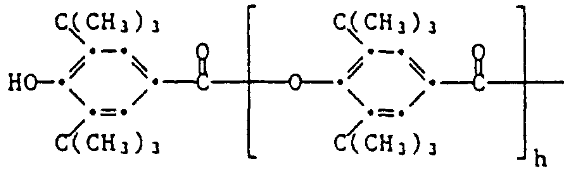 mit h = 0 oder 1;

oder $R_3$ und $R_4$ sind zusammen Alkylen mit 4 bis 6 Kohlenstoffatomen oder 2-Oxoalkylen oder der cyclische Acylrest einer aliphatischen oder aromatischen 1,2- oder 1,3-Dicarbonsäure,
wenn p = 2 ist,
ist $R_4$ $C_2$-$C_{12}$-Alkylen, $C_6$-$C_{12}$-Arylen, Xylylen, eine Gruppe $-CH_2CH(OH)-CH_2-$ oder eine Gruppe $-CH_2-CH(OH)-CH_2-O-X-O-CH_2-CH(OH)-CH_2-$, worin X ist $C_2$-$C_{10}$-Alkylen, $C_6$-$C_{15}$-Arylen oder $C_6$-$C_{12}$-Cycloalkylen; oder, vorausgesetzt, daß $R_3$ nicht Alkanoyl, Alkenoyl oder Benzoyl ist, kann $R_4$ auch ein zweiwertiger Acylrest einer aliphatischen, cycloaliphatischen oder aromatischen Dicarbonsäure oder Dicarbamidsäure sein, oder kann die Gruppe -CO- sein, oder $R_4$ ist eine Gruppe der Formel II

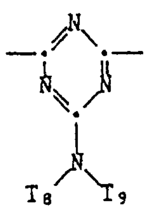 (II)

worin $T_8$ und $T_9$ unabhängig Wasserstoff, Alkyl mit 1 bis 18 Kohlenstoffatomen oder eine Gruppe der Formel I sind, oder $T_8$ und $T_9$ sind zusammen Alkylen mit 4 bis 6 Kohlenstoffatomen oder 3-Oxapentamethylen;
wenn p = 3 ist,
ist $R_4$ 2,4,6-Triazintriyl,
n = 1 oder 2, und
wenn n = 1 ist,
sind $R_5$ und $R'_5$ unabhängig $C_1$-$C_{12}$-Alkyl, $C_2$-$C_{12}$-Alkenyl, $C_7$-$C_{12}$-Aralkyl, oder $R_5$ ist auch Wasserstoff, oder $R_5$ und $R'_5$ sind zusammen $C_2$-$C_8$-Alkylen oder Hydroxyalkylen oder $C_4$-$C_{22}$-Acyloxyalkylen;
wenn n = 2 ist,
sind $R_5$ und $R'_5$ zusammen $(-CH_2)_2C(CH_2-)_2$;
$R_6$ ist Wasserstoff, $C_1$-$C_{12}$-Alkyl, Allyl, Benzyl, Glycidyl oder $C_2$-$C_6$-Alkoxyalkyl;
wenn n = 1 ist,
ist $R_7$ Wasserstoff, $C_1$-$C_{12}$-Alkyl, $C_3$-$C_5$-Alkenyl, $C_7$-$C_9$-Aralkyl, $C_5$-$C_7$-Cycloalkyl, $C_2$-$C_4$-Hydroxyalkyl, $C_2$-$C_6$-Alkoxyalkyl, $C_6$-$C_{10}$-Aryl, Glycidyl, eine Gruppe der Formel $-(CH_2)_t-COO-Q$ oder der Formel $-(CH_2)_t-O-CO-Q$, worin t = 1 oder 2 ist und Q ist $C_1$-$C_4$-Alkyl oder Phenyl; oder
wenn n = 2 ist,
ist $R_7$ $C_2$-$C_{12}$-Alkylen, $C_6$-$C_{12}$-Arylen, eine Gruppe $-CH_2CH(OH)-CH_2-O-X-O-CH_2-CH(OH)-CH_2-$, worin X $C_2$-$C_{10}$-Alkylen, $C_6$-$C_{15}$-Arylen oder $C_6$-$C_{12}$-Cycloalkylen ist,
oder eine Gruppe $-CH_2CH(OZ')CH_2-(OCH_2-CH(OZ')CH_2)_2-$ ist, worin Z' Wasserstoff, $C_1$-$C_{18}$-Alkyl, Allyl, Benzyl, $C_2$-$C_{12}$-Alkanoyl oder Benzoyl ist;
$Q_1$ ist $-N(R_8)-$ oder -O-;
E ist $C_1$-$C_3$-Alkylen, die Gruppe $-CH_2-CH(R_9)-O-$, worin $R_9$ für Wasserstoff, Methyl oder Phenyl steht, die Gruppe $-(CH_2)_3-NH-$ oder eine direkte Bindung;
$R_{10}$ ist Wasserstoff oder $C_1$-$C_{18}$-Alkyl,
$R_8$ ist Wasserstoff, $C_1$-$C_{18}$-Alkyl, $C_5$-$C_7$-Cycloalkyl, $C_7$-$C_{12}$-Aralkyl, Cyanoethyl, $C_6$-$C_{10}$-Aryl, die Gruppe $-CH_2-CH(R_9)-OH$, worin $R_9$ die vorstehend definierte Bedeutung hat; oder $R_8$ ist eine Gruppe der Formel I oder eine Gruppe der Formel

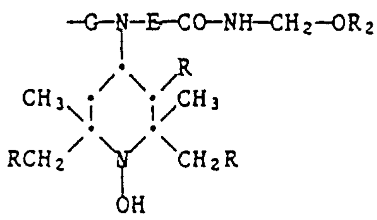

worin G $C_2$-$C_6$-Alkylen oder $C_6$-$C_{12}$-Arylen ist, oder
$R_8$ ist eine Gruppe -E-CO-NH-$CH_2$-$OR_{10}$;
$T_3$ ist Ethylen oder 1,2-Propylen, oder ist die wiederkehrende Struktureinheit abgeleitet von einem alpha-Olefincopolymeren mit einem Alkylacrylat oder -methacrylat;
k ist 2 bis 100;
$T_4$ hat die gleiche Bedeutung wie $R_4$, wenn p = 1 oder 2 ist,
$T_5$ ist Methyl,
$T_6$ ist Methyl oder Ethyl, oder $T_5$ und $T_6$ sind zusammen Tetramethylen oder Pentamethylen;
M und Y sind unabhängig voneinander Methylen oder Carbonyl; $T_7$ ist dasselbe wie $R_7$;
$T_{10}$ und $T_{11}$ sind unabhängig voneinander Alkylen mit 2 bis 12 Kohlenstoffatomen, oder $T_{11}$ ist eine Gruppe der Formel II;
e = 2, 3 oder 4, und
$T_{12}$ ist eine Gruppe -N($R_5$)-($CH_2$)$_d$-N($R_5$)-oder

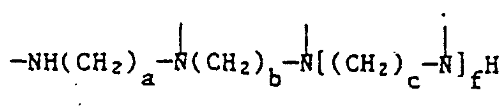

wobei a, b und c unabhängig voneinander 2 oder 3, d = 2 bis 10 und f = 0 oder 1 sind;
$T_{13}$ ist dasselbe wie $R_4$ mit der Maßgabe, daß $T_{13}$ nicht Wasserstoff sein kann, wenn n = 1 ist;
$E_1$ und $E_2$, die verschieden sind, sind jeweils -CO- oder -N($E_5$)-, und $E_5$ ist Wasserstoff, $C_1$-$C_{12}$-Alkyl oder $C_4$-$C_{22}$-Alkoxycarbonylalkyl;
$E_3$ ist Wasserstoff, Alkyl mit 1 bis 30 Kohlenstoffatomen, Phenyl, Naphthyl, wobei dieses Phenyl oder dieses Naphthyl substituiert ist durch Chlor oder durch Alkyl mit 1 bis 4 Kohlenstoffatomen, oder Phenylalkyl mit 7 bis 12 Kohlenstoffatomen, oder dieses Phenylalkyl substituiert durch Alkyl mit 1 bis 4 Kohlenstoffatomen, und
$E_4$ ist Wasserstoff, Alkyl mit 1 bis 30 Kohlenstoffatomen, Phenyl, Naphthyl oder Phenylalkyl mit 7 bis 12 Kohlenstoffatomen, oder
$E_3$ und $E_4$ sind zusammen Polymethylen mit 4 bis 17 Kohlenstoffatomen, oder dieses Polymethylen substituiert durch bis zu vier Alkylgruppen mit 1 bis 4 Kohlenstoffatomen.

**3.** Zusammensetzung gemäß Anspruch 2, welche ein gehindertes Aminderivat der Formel A, B, G-K oder M enthält, worin R Wasserstoff ist und $T_5$ und $T_6$ sind Methyl.

**4.** Zusammensetzung gemäß Anspruch 2, welche ein gehindertes Aminderivat der Formel A enthält, worin R Wasserstoff ist, m = 1, 2 oder 4 und wenn m = 1 ist, ist $R_1$ ein Acylrest einer aliphatischen Carboxylsäure mit 12 bis 18 Kohlenstoffatomen oder einer aromatischen Carboxylsäure mit 7 bis 15 Kohlenstoffatomen, und wenn m = 2 ist, ist $R_1$ ein zweiwertiger Acylrest einer aliphatischen Dicarbonsäure mit 4 bis 18 Kohlenstoffatomen oder einer aromatischen Dicarbonsäure mit 8 bis 14. Kohlenstoffatomen, oder $R_2$ ist eine Gruppe

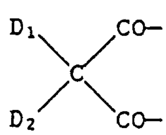

worin $D_1$ $C_1$-$C_8$-Alkyl oder 3,4-Di-tert-butyl-4-hydroxybenzyl ist und $D_2$ ist $D_1$ oder Wasserstoff, und wenn m = 4

ist, ist $R_1$ ein vierwertiger Acylrest einer Butan- oder Pentantetracarbonsäure.

**5.** Zusammensetzung gemäß Anspruch 1, enthaltend Di-(1-hydroxy-2,2,6,6-tetramethylpiperidin-4-yl)-sebacat.

**6.** Zusammensetzung gemäß Anspruch 1, enthaltend 1-Hydroxy-2,2,6,6-tetramethylpiperidin-4-yl)-stearat.

**7.** Zusammensetzung gemäß Anspruch 1, enthaltend Di-(1-hydroxy-2,2,6,6-tetramethylpiperidin-4-yl)-diethylmalonat.

**8.** Zusammensetzung gemäß Anspruch 1, enthaltend Di-(1-hydroxy-2,2,6,6-tetramethylpiperidin-4-yl)-isophthalat.

**9.** Zusammensetzung gemäß Anspruch 1, enthaltend Tetrakis-(1-hydroxy-2,2,6,6-tetramethyl-4-piperidyl)-1,2,3,4-butantetracarboxylat.

**10.** Zusammensetzung gemäß Anspruch 1, enthaltend das Polykondensationsprodukt von 2,4-Dichlor-6-tert-octylamino-striazin und 4,4'-Hexamethylenbis-(amino-1-hydroxy-2,2,6,6-tetramethylpiperidin).

**11.** Zusammensetzung gemäß Anspruch 1, worin das gehinderte Aminderivat in einer Menge von 0,1 bis 10 Gew.-%, basierend auf den Harzfeststoffen, enthalten ist.

**12.** Zusammensetzung gemäß Anspruch 1, welche zusätzlich einen UV-Absorber enthält, ausgewählt aus der Gruppe bestehend aus Benzophenonen, Benztriazolen, Acrylsäurederivaten, Aryls-triazinen, organischen Nickelverbindungen und Oxaniliden.

**13.** Zusammensetzung gemäß Anspruch 12, welche einen Benzotriazol-UV-Absorber enthält, ausgewählt aus der Gruppe bestehend aus 2-[2-Hydroxy-3,5-di-(alpha,alpha-dimethylbenzyl)-phenyl]-2H-benzotriazol, 2-(2-Hydroxy-3,5-di-tert-octylphenyl)-2H-benzotriazol, 2-(2-Hydroxy-3-alpha,alpha-dimethylbenzyl-5-tert-octylphenyl)-2H-benzotriazol, 2-(2-Hydroxy-3-tert-octyl-5-alpha, alpha-dimethylbenzylphenyl)-2H-benzotriazol, 2-(2-Hydroxy-5-tert-octylphenyl)-2H-benzotriazol, 2-[2-Hydroxy-3-tert-butyl-5-(2-(omega-hydroxy-octa-(ethylenoxy)-carbonyl)-ethylphenyl]-2H-benzotriazol, 2-(2-Hydroxy-3,5-di-tert-amylphenyl)-2H-benzotriazol, 5-Chlor-2-[2-hydroxy-3,5-di-(alpha,alpha-dimethylbenzyl)-phenyl]-2H-benzotriazol, 5-Chlor-2-(2-hydroxy-3,5-di-tert-butylphenyl)-2H-benzotriazol, 2-[2-Hydroxy-3-tert-butyl-5-(2-octyloxycarbonylethyl)-phenyl]-5-chlor-2H-benzotriazol, 2-(2-Hydroxy-3-sec-dodecyl-5-methylphenyl)-2H-benzotriazol und Hexamethylen-di[β-(3-tert-butyl-4-hydroxy-4-[2-benzotriazolyl]-phenyl)-propionat].

**14.** Überzugszusammensetzung gemäß Anspruch 1, welche ein bei gewöhnlicher Temperatur härtbares System ist, basierend auf einem Alkydharz, thermoplastischen Acrylharz, Acrylalkydharz, Polyesterharz, oder diese Harze modifiziert mit Silikonen, Isocyanaten, Epoxiden, Isocyanuraten, Ketiminen oder Oxazolidinen, oder das System basiert auf einem Celluloseester oder auf einem Epoxidharz.

**15.** Überzugszusammensetzung gemäß Anspruch 1, welche ein säurekatalysiertes wärmehärtbares System ist, basierend auf einem heizvernetzbaren Acryl-, Polyester-, Polyurethan-, Polyamid- oder Alkydharz.

**16.** Zusammensetzung gemäß Anspruch 1, welche ein Lack für industrielle Oberflächenlacke ist.

**17.** Zusammensetzung gemäß Anspruch 1, welche ein Nacharbeitungslack für Automobile ist.

**18.** Verbindung entsprechend den Formeln A' oder B'

(A')

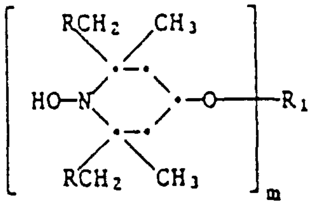

(B')

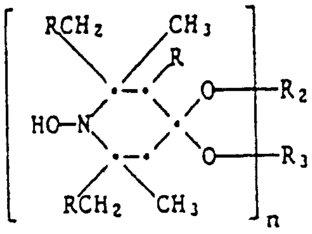

worin R = Wasserstoff oder Methyl,
m ist 1 bis 4;
wenn m = 1 ist, ist $R_1$

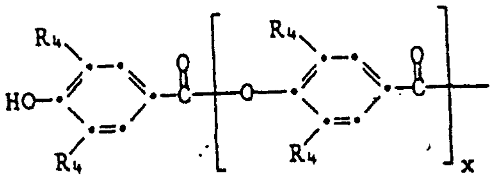

worin x = 0 oder 1 ist, und $R_4$ ist unabhängig $C_1$-$C_8$-Alkyl, oder

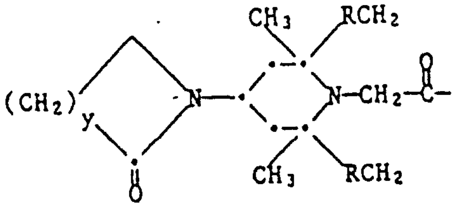

mit y = 2 bis 4;
wenn m = 2 ist, ist $R_1$

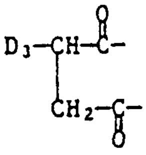

oder

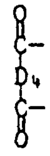

worin
$D_3$ ist $C_1$-$C_{18}$-Alkyl oder $C_2$-$C_{18}$-Alkenyl, und $D_4$ ist $C_5$-$C_{12}$-Cycloalkylen;
wenn m = 3 ist, ist $R_1$ ein dreiwertiger Acylrest einer aliphatischen, ungesättigten aliphatischen, cycloaliphatischen oder aromatischen Tricarbonsäure mit bis zu 20 Kohlenstoffatomen;
wenn m = 4 ist, ist $R_1$ ein vierwertiger Acylrest einer gesättigten oder ungesättigten aliphatischen oder aromatischen Tetracarbonsäure mit bis zu 20 Kohlenstoffatomen;
n ist 1
wenn n = 1 ist, sind $R_2$ und $R_3$ unabhängig Wasserstoff, $C_1$-$C_{12}$-Alkyl, $C_2$-$C_{12}$-Alkenyl oder $C_7$-$C_{12}$-Aralkyl.

**19.** Verbindung gemäß Anspruch 18, worin R Wasserstoff ist.

**20.** Verbindung der Formel A' gemäß Anspruch 18, worin R für Wasserstoff steht, m = 1, 2 oder 4 ist, und wenn m = 1 ist, ist $R_1$ eine Gruppe

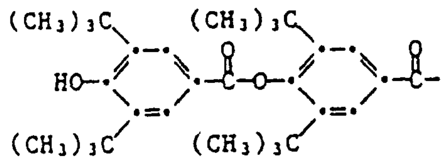

oder

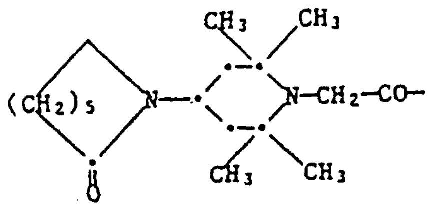

wenn m = 2 ist, ist $R_1$ eine Gruppe

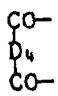

worin
$D_4$ Cyclohexylen ist und
wenn m = 4 ist, ist $R_1$ der vierwertige Acylrest einer Butantetracarbonsäure.

**21.** Di-(1-hydroxy-2,2,6,6-tetramethylpiperidin-4-yl)-methylmalonat.

**22.** Di-(1-hydroxy-2,2,6,6-tetramethylpiperidin-4-yl)-n-butylmalonat.

**23.** Di-(1-hydroxy-2,2,6,6-tetramethylpiperidin-4-yl)-diethylmalonat.

**24.** Di-(1-hydroxy-2,2,6,6-tetramethylpiperidin-4-yl) 2-(4-hydroxy-3,5-di-tert-butylbenzyl)-2-n-butylmalonat.

**25.** Tetrakis-(1-hydroxy-2,2,6,6-tetramethyl-4-piperidyl)-1,2,3,4-butantetracarboxylat gemäß Anspruch 18.

**26.** Verwendung einer Verbindung von Anspruch 18 als Stabilisator für organische Materialien gegen aktinischen Abbau.

**27.** Verwendung gemäß Anspruch 26 als Stabilisator für organische Polymere.

**28.** Verwendung gemäß Anspruch 26 als Stabilisator für photographische Schichten.

**Revendications**

**1.** Composition de revêtement durcissante dans les conditions ambiantes ou thermodurcissable avec un catalyseur

acide, contenant une quantité stabilisante efficace d'un composé d'amine encombrée substituée par hydroxyle et contenant le groupe de formule

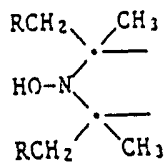

dans laquelle R est un atome d'hydrogène ou un groupe méthyle.

2. Composition selon la revendication 1, contenant un dérivé d'amine encombrée correspondant à l'une des formules A à M

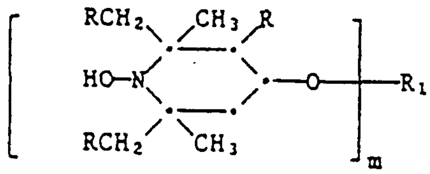

(A)

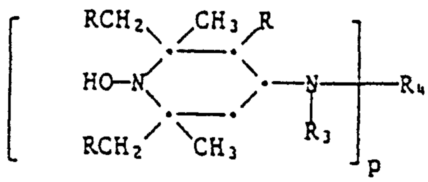

(B)

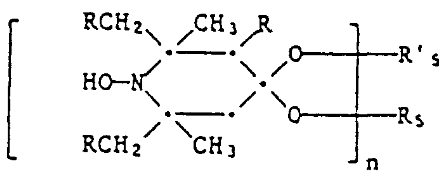

(C)

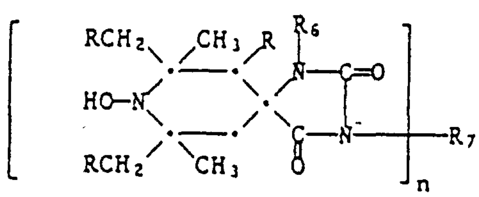

(D)

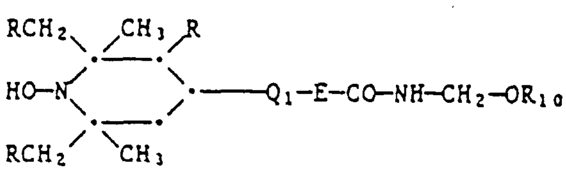

(E)

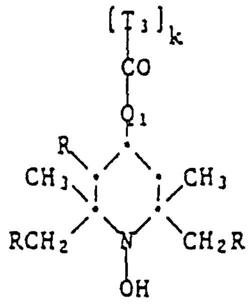

(F)

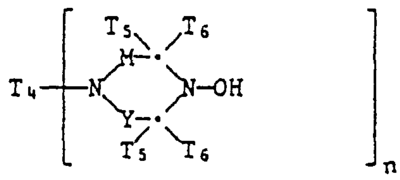

(G)

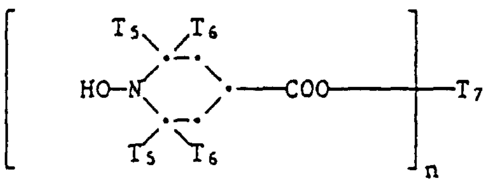

(H)

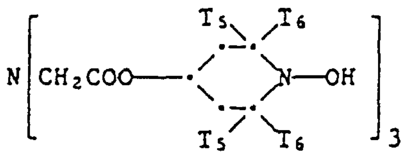

(I)

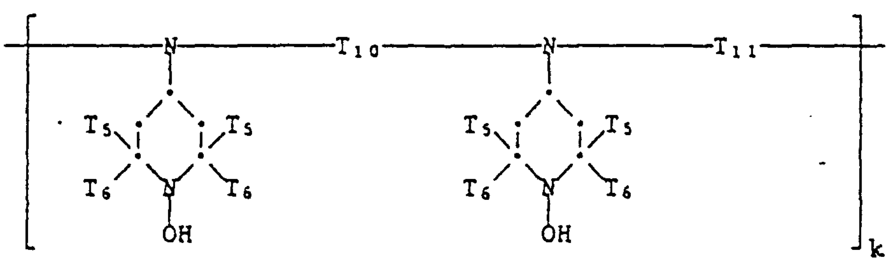

(J)

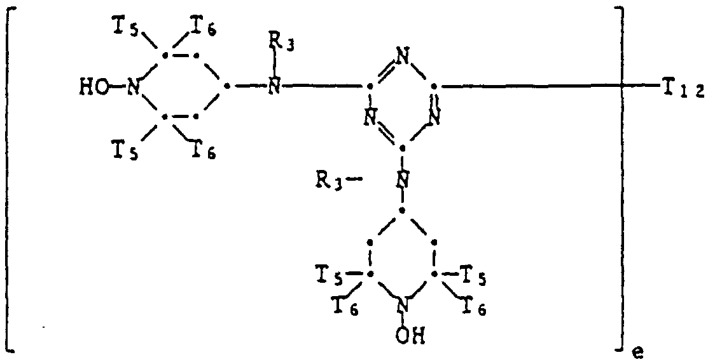

(K)

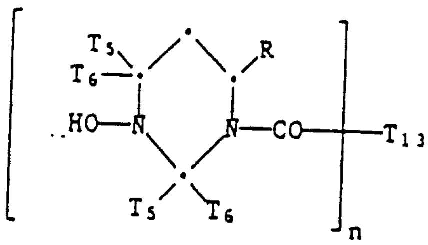

(L)

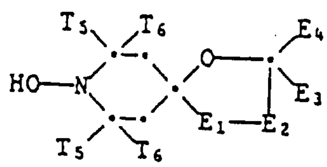

(M)

dans lesquelles
R est un atome d'hydrogène ou un groupe méthyle,
m est 1-4,
lorsque m est 1,
$R_1$ est un atome d'hydrogène, un groupe alkyle en $C_1$-$C_{18}$ éventuellement interrompu par un ou plusieurs atomes d'oxygène, alcényle en $C_2$-$C_{12}$, aryle en $C_6$-$C_{10}$, aralkyle en $C_7$-$C_{12}$, glycidyle, un radical acyle monovalent d'un acide carboxylique ou carbamique aliphatique, cycloaliphatique, araliphatique ou aromatique; ou $R_1$ est un groupe

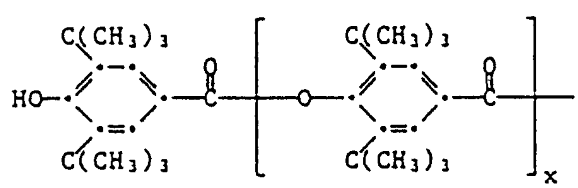

dans lequel x est 0 ou 1, ou est un groupe

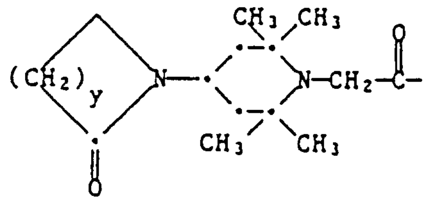

dans lequel y est 2-4;

lorsque m est 2,

$R_1$ est un groupe alkylène en $C_1$-$C_{12}$, alcénylène en $C_4$-$C_{12}$, xylylène, un radical acyle divalent d'un acide dicarboxylique ou dicarbamique aliphatique, cycloaliphatique, araliphatique ou aromatique; ou $R_1$ est un groupe

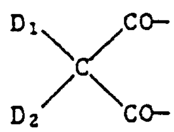 ou 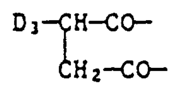

dans lesquels $D_1$ et $D_2$ sont indépendamment un atome d'hydrogène, un groupe alkyle en $C_1$-$C_8$, phényle, benzyle ou 3,5-di-t-butyl-4-hydroxybenzyle, et $D_3$ est un groupe alkyle ou alcényle contenant jusqu'à 18 atomes de carbone;

lorsque m est 3,

$R_1$ est un radical acyle trivalent d'un acide tricarboxylique aliphatique, aliphatique insaturé, cycloaliphatique ou aromatique;

lorsque m est 4,

$R_1$ est un radical acyle tétravalent d'un acide tétracarboxylique aliphatique saturé ou insaturé ou aromatique;

p est 1, 2 ou 3,

$R_3$ est un atome d'hydrogène ou un groupe alkyle en $C_1$-$C_{12}$, cycloalkyle en $C_5$-$C_7$, aralkyle en $C_7$-$C_9$, alcanoyle en $C_2$-$C_{18}$, alcénoyle en $C_3$-$C_5$ ou benzoyle;

lorsque p est 1,

$R_4$ est un atome d'hydrogène, un groupe alkyle en $C_1$-$C_{18}$, cycloalkyle en $C_5$-$C_7$, alcényle en $C_2$-$C_8$ non substitué ou substitué par un groupe cyano, carbonyle ou carbamide, aryle, aralkyle, ou est un groupe glycidyle, un groupe de formule $-CH_2-CH(OH)-Z$ ou -CONH-Z où Z est un atome d'hydrogène ou un groupe méthyle ou phényle; ou $R_4$ est un groupe de formule (I)

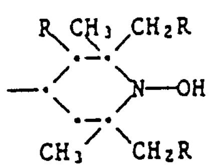 (I)

ou un groupe de formule

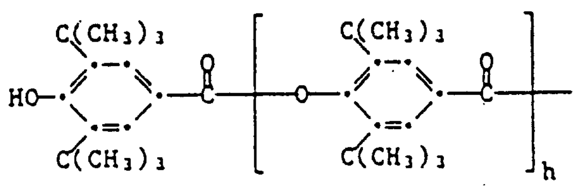 h étant 0 ou 1;

ou $R_3$ et $R_4$ forment ensemble un groupe alkylène de 4 à 6 atomes de carbone ou 2-oxoalkylène ou le radical acyle cyclique d'un acide 1,2- ou 1,3-dicarboxylique aliphatique ou aromatique;

lorsque p est 2,
$R_4$ est un groupe alkylène en $C_2$-$C_{12}$, arylène en $C_6$-$C_{12}$, xylylène, un groupe $-CH_2CH(OH)-CH_2-$ ou un groupe $-CH_2-CH(OH)-CH_2-O-X-O-CH_2-CH(OH)-CH_2-$ dans lequel X est un groupe alkylène en $C_2$-$C_{10}$, arylène en $C_6$-$C_{15}$ ou cycloalkylène en $C_6$-$C_{12}$; ou, sous réserve que $R_3$ ne soit pas un groupe alcanoyle, alcénoyle ou benzoyle, $R_4$ peut aussi être un radical acyle divalent d'un acide dicarboxylique ou dicarbamique aliphatique, cycloaliphatique ou aromatique, ou peut être le groupe -CO-, ou
$R_4$ est un groupe de formule (II)

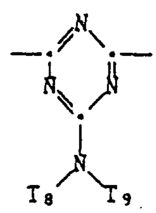

(II)

dans laquelle $T_8$ et $T_9$ sont indépendamment un atome d'hydrogène, un groupe alkyle de 1 à 18 atomes de carbone ou un groupe de formule I, ou $T_8$ et $T_9$ forment ensemble un groupe alkylène de 4 à 6 atomes de carbone ou 3-oxapentaméthylène;
lorsque p est 3,
$R_4$ est le radical 2,4,6-triazinetriyle,
n est 1 ou 2 et
lorsque n est 1,
$R_5$ et $R_5'$ sont indépendamment un groupe alkyle en $C_1$-$C_{12}$, alcényle en $C_2$-$C_{12}$, aralkyle en $C_7$-$C_{12}$, ou $R_5$ est aussi un atome d'hydrogène, ou $R_5$ et $R_5'$ forment ensemble un groupe alkylène ou hydroxyalkylène en $C_2$-$C_8$ ou acyloxyalkylène en $C_4$-$C_{22}$;
lorsque n est 2,
$R_5$ et $R_5'$ forment ensemble le groupe $(-CH_2)_2C(CH_2-)_2$;
$R_6$ est un atome d'hydrogène ou un groupe alkyle en $C_1$-$C_{12}$, allyle, benzyle, glycidyle ou alcoxyalkyle en $C_2$-$C_6$;
lorsque n est 1,
$R_7$ est un atome d'hydrogène, un groupe alkyle en $C_1$-$C_{12}$, alcényle en $C_3$-$C_5$, aralkyle en $C_7$-$C_9$, cycloalkyle en $C_5$-$C_7$, hydroxyalkyle en $C_2$-$C_4$, alcoxyalkyle en $C_2$-$C_6$, aryle en $C_6$-$C_{10}$, glycidyle, un groupe de formule $-(CH_2)_t-COO-Q$ ou de formule $-(CH_2)_t-O-CO-Q$ où t est 1 ou 2 et Q est un groupe alkyle en $C_1$-$C_4$ ou phényle; ou
lorsque n est 2,
$R_7$ est un groupe alkylène en $C_2$-$C_{12}$, arylène en $C_6$-$C_{12}$, un groupe $-CH_2-CH(OH)-CH_2-O-X-O-CH_2-CH(OH)-CH_2-$ dans lequel X est un radical alkylène en $C_2$-$C_{10}$, arylène en $C_6$-$C_{15}$ ou cycloalkylène en $C_6$-$C_{12}$, ou un groupe $-CH_2-CH(OZ')-CH_2-(OCH_2-CH(OZ')-CH_2)_2-$ dans lequel Z' est un atome d'hydrogène ou un groupe alkyle en $C_1$-$C_{18}$, allyle, benzyle, alcanoyle en $C_2$-$C_{12}$ ou benzoyle;
$Q_1$ est $-N(R_8)-$ ou -O-;
E est un groupe alkylène en $C_1$-$C_3$, le groupe $-CH_2-CH(R_9)-O-$ dans lequel $R_9$ est l'atome d'hydrogène, un groupe méthyle ou phényle, le groupe $-(CH_2)_3-NH-$ ou une liaison directe;
$R_{10}$ est un atome d'hydrogène ou un groupe alkyle en $C_1$-$C_{18}$;
$R_8$ est un atome d'hydrogène, un groupe alkyle en $C_1$-$C_{18}$, cycloalkyle en $C_5$-$C_7$, aralkyle en $C_7$-$C_{12}$, cyanoéthyle, aryle en $C_6$-$C_{10}$, le groupe $-CH_2-CH(R_9)-OH$ dans lequel $R_9$ a la signification définie ci-dessus; ou $R_8$ est un groupe de formule I ou un groupe de formule

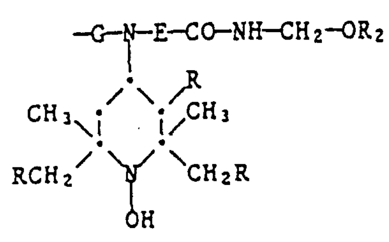

dans laquelle G est un groupe alkylène en $C_2$-$C_6$ ou arylène en $C_6$-$C_{12}$, ou $R_8$ est un groupe $-E-CO-NH-CH_2-OR_{10}$;

$T_3$ est un groupe éthylène ou 1,2-propylène, ou est l'unité constitutive répétitive d'un copolymère d'$\alpha$-oléfine avec un acrylate ou un méthacrylate d'alkyle;
k est compris entre 2 et 100;
$T_4$ a la même signification que $R_4$ lorsque p est 1 ou 2;
$T_5$ est un groupe méthyle;
$T_6$ est un groupe méthyle ou éthyle, ou $T_5$ et $T_6$ forment ensemble un groupe tétraméthylène ou pentaméthylène;
M et Y sont indépendamment un groupe méthylène ou carbonyle;
$T_7$ est identique à $R_7$;
$T_{10}$ et $T_{11}$ sont indépendamment un groupe alkylène de 2 à 12 atomes de carbone, ou $T_{11}$ est un groupe de formule II;
e est 2, 3 ou 4 et
$T_{12}$ est un groupe -N($R_5$)-($CH_2$)$_d$-N($R_5$)- ou

$$-NH(CH_2)_a-\overset{|}{N}(CH_2)_b-\overset{|}{N}[(CH_2)_c-\overset{|}{N}]_fH$$

où a, b et c sont indépendamment 2 ou 3, d est compris entre 2 et 10 et f est 0 ou 1;
$T_{13}$ est identique à $R_4$, sous réserve que $T_{13}$ ne puisse pas être l'hydrogène quand n est 1;
$E_1$ et $E_2$ sont différents et sont chacun -CO- ou -N($E_5$)-, et $E_5$ est un atome d'hydrogène ou un groupe alkyle en $C_1$-$C_{12}$ ou alcoxycarbonylalkyle en $C_4$-$C_{22}$;
$E_3$ est un atome d'hydrogène, un groupe alkyle de 1 à 30 atomes de carbone, phényle, naphtyle, ledit phényle ou ledit naphtyle substitué par du chlore ou par alkyle de 1 à 4 atomes de carbone, ou phénylalkyle de 7 à 12 atomes de carbone, ou ledit phénylalkyle substitué par alkyle de 1 à 4 atomes de carbone, et
$E_4$ est un atome d'hydrogène ou un groupe alkyle de 1 à 30 atomes de carbone, phényle, naphtyle ou phénylalkyle de 7 à 12 atomes de carbone, ou
$E_3$ et $E_4$ forment ensemble un polyméthylène de 4 à 17 atomes de carbone, ou ledit polyméthylène substitué par 1 à 4 groupes alkyle de 1 à 4 atomes de carbone.

**3.** Composition selon la revendication 2, qui contient un dérivé d'amine encombrée de formule A, B, G-K ou M, dans lesquelles R est un atome d'hydrogène et $T_5$ et $T_6$ sont des groupes méthyle.

**4.** Composition selon la revendication 2, qui contient un dérivé d'amine encombrée de formule A dans laquelle R est un atome d'hydrogène, m est 1, 2 ou 4 et, lorsque m est 1, $R_1$ est un radical acyle d'un acide carboxylique aliphatique ayant 12 à 18 atomes de carbone ou d'un acide carboxylique aromatique ayant 7 à 15 atomes de carbone et, lorsque m est 2, $R_1$ est un radical acyle divalent d'un acide dicarboxylique aliphatique ayant 4 à 18 atomes de carbone ou d'un acide dicarboxylique aromatique ayant 8 à 14 atomes de carbone ou $R_1$ est un groupe

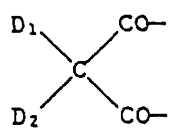

dans lequel $D_1$ est un groupe alkyle en $C_1$-$C_8$ ou 3,4-di-tert-butyl-4-hydroxybenzyle et $D_2$ est $D_1$ ou un atome d'hydrogène, et, lorsque m est 4, $R_1$ est un radical acyle tétravalent d'un acide butane- ou pentanetétracarboxylique.

**5.** Composition selon la revendication 1, contenant du sébacate de di(1-hydroxy-2,2,6,6-tétraméthylpipéridine-4-yle).

**6.** Composition selon la revendication 1, contenant du stéarate de 1-hydroxy-2,2,6,6-tétraméthylpipéridine-4-yle.

**7.** Composition selon la revendication 1, contenant du diéthylmalonate de di(1-hydroxy-2,2,6,6-tétraméthylpipéridine-4-yle).

**8.** Composition selon la revendication 1, contenant de l'isophtalate de di(1-hydroxy-2,2,6,6-tétraméthylpipéridine-4-yle).

**9.** Composition selon la revendication 1, contenant du 1,2,3,4-butanetétracarboxylate de tétrakis(1-hydroxy-2,2,6,6-tétraméthylpipéridine-4-yle).

**10.** Composition selon la revendication 1, contenant le produit de polycondensation de la 2,4-dichloro-6-tert-octylamino-s-triazine et de la 4,4'-hexaméthylènebis(amino-1-hydroxy-2,2,6,6-tétraméthylpipéridine).

**11.** Composition selon la revendication 1, dans laquelle le dérivé d'amine encombrée est contenu en une quantité de 0,1 à 10 % en masse par rapport aux matières solides de la résine.

**12.** Composition selon la revendication 1, qui contient en outre un absorbant d'UV choisi dans le groupe constitué par des benzophénones, des benzotriazoles, des dérivés de l'acide acrylique, des aryl-s-triazines, des composés organiques du nickel et des oxanilides.

**13.** Composition selon la revendication 12, qui contient un absorbant d'UV de type benzotriazole choisi dans le groupe constitué par le 2-[2-hydroxy-3,5-di(α,α-diméthylbenzyl)phényl]-2H-benzotriazole, le 2-(2-hydroxy-3,5-di-tert-octylphényl)-2H-benzotriazole, le 2-(2-hydroxy-3-α,α-diméthylbenzyl-5-tert-octylphényl)-2H-benzotriazole, le 2-(2-hydroxy-3-tert-octyl-5-α,α-diméthylbenzylphényl)-2H-benzotriazole, le 2-(2-hydroxy-5-tert-octylphényl)-2H-benzotriazole, le 2-[2-hydroxy-3-tert-butyl-5-(2-(ω-hydroxyocta(éthylèneoxy)-carbonyl)éthyl)phényl]-2H-benzotriazole, le 2-(2-hydroxy-3,5-di-tert-amylphényl)-2H-benzotriazole, le 5-chloro-2-[2-hydroxy-3,5-di(α,α-diméthylbenzyl)phényl]-2H-benzotriazole, le 5-chloro-2-(2-hydroxy-3,5-di-tert-butylphényl)-2H-benzotriazole, le 2-[2-hydroxy-3-tert-butyl-5-(2-octyloxycarbonyléthyl)phényl]-5-chloro-2H-benzotriazole, le 2-(2-hydroxy-3-sec-dodécyl-5-méthylphényl)-2H-benzotriazole et le di[β-(3-tert-butyl-4-hydroxy-5-[2-benzotriazolyl]phényl)propionate] d'hexaméthylène.

**14.** Composition de revêtement selon la revendication 1, qui est un système durcissable dans les conditions ambiantes à base d'une résine alkyde, d'une résine acrylique thermoplastique, d'une résine alkyde acrylique, d'une résine polyester, ou desdites résines modifiées avec des silicones, des isocyanates, des époxydes, des isocyanurates, des cétimines ou des oxazolidines, ou bien le système est à base d'un ester de cellulose ou d'une résine époxy.

**15.** Composition de revêtement selon la revendication 1, qui est un système thermodurcissable catalysé par un acide, à base d'une résine acrylique, polyester, polyuréthane, polyamide ou alkyde réticulable à chaud.

**16.** Composition selon la revendication 1, qui est un émail pour des couches de finition industrielles.

**17.** Composition selon la revendication 1, qui est un émail de retouches pour l'automobile.

**18.** Composé correspondant aux formules A' ou B'

(A')

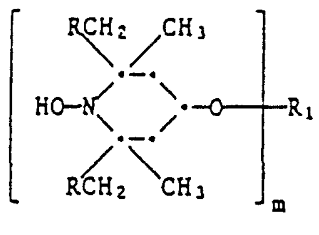

(B')

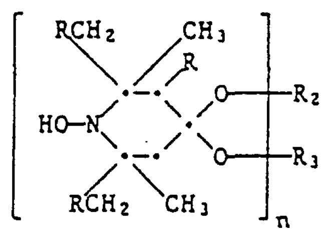

dans lesquelles R est un atome d'hydrogène ou un groupe méthyle;
m est 1-4;
lorsque m est 1, $R_1$ est

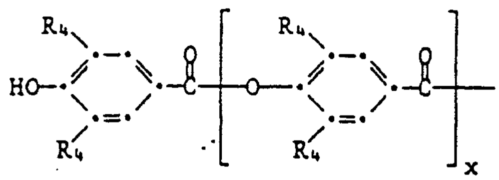

où x est 0 ou 1 et les $R_4$ sont indépendamment des groupes alkyle en $C_1$-$C_8$, ou

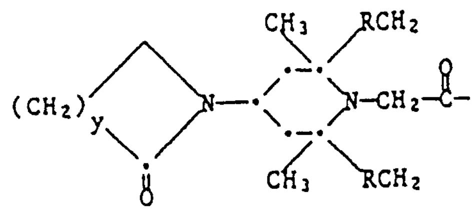

où y est 2-4;
lorsque m est 2, $R_1$ est

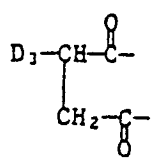

ou

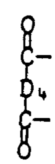

où $D_3$ est un groupe alkyle en $C_1$-$C_{18}$ ou alcényle en $C_2$-$C_{18}$, et $D_4$ est un groupe cycloalkylène en $C_5$-$C_{12}$;
lorsque m est 3,
$R_1$ est un radical acyle trivalent d'un acide tricarboxylique aliphatique, aliphatique insaturé, cycloaliphatique ou aromatique ayant jusqu'à 20 atomes de carbone;
lorsque m est 4,
$R_1$ est un radical acyle tétravalent d'un acide tétracarboxylique aliphatique saturé ou insaturé ou aromatique ayant jusqu'à 20 atomes de carbone;
n est 1 ;
lorsque n est 1,
$R_2$ et $R_3$ sont indépendamment un atome d'hydrogène ou un groupe alkyle en $C_1$-$C_{12}$, alcényle en $C_2$-$C_{12}$ ou aralkyle en $C_7$-$C_{12}$.

**19.** Composé selon la revendication 18, dans lequel R est un atome d'hydrogène.

**20.** Composé de formule A' selon la revendication 18, dans lequel
R est un atome d'hydrogène, m est 1, 2 ou 4 et
lorsque m est 1,
$R_1$ est un groupe

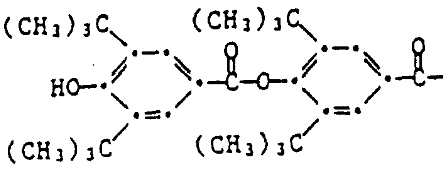

ou

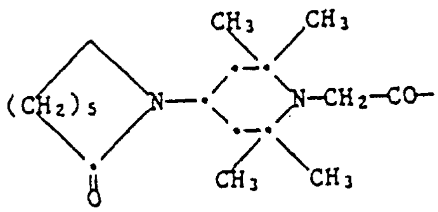

lorsque m est 2,
$R_1$ est un groupe

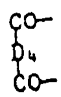

dans lequel $D_4$ est un groupe cyclohexylène,
et lorsque m est 4,
$R_1$ est le radical acyle tétravalent d'un acide butanetétracarboxylique.

**21.** Méthylmalonate de di(1-hydroxy-2,2,6,6-tétraméthylpipéridine-4-yle).

**22.** n-Butylmalonate de di(1-hydroxy-2,2,6,6-tétraméthylpipéridine-4-yle).

**23.** Diéthylmalonate de di(1-hydroxy-2,2,6,6-tétraméthylpipéridine-4-yle).

**24.** 2-(4-Hydroxy-3,5-di-tert-butylbenzyl)-2-n-butylmalonate de di(1-hydroxy-2,2,6,6-tétraméthylpipéridine-4-yle).

**25.** 1,2,3,4-Butanetétracarboxylate de tétrakis(1-hydroxy-2,2,6,6-tétraméthylpipéridine-4-yle) selon la revendication 18.

**26.** Utilisation d'un composé selon la revendication 18 comme stabilisant de matières organiques contre la dégradation actinique.

**27.** Utilisation selon la revendication 26 comme stabilisant pour des polymères organiques.

**28.** Utilisation selon la revendication 26 comme stabilisant pour des couches photographiques.